# EUROPEAN PATENT APPLICATION

(11) **EP 4 523 759 A2**
(43) Date of publication of application: **19.03.2025**
(21) Application number: 24218475.2
(22) Date of filing: 19.05.2016
(51) Int. Cl.: A61P 43/00

(54) **B-CELL DEPLETION AS A DIAGNOSTIC MARKER**

(30) Priority: 20.05.2015 US 201562164278 P
(62) Divisional of application: 16728620.2
(71) Applicant: Amgen Research (Munich) GmbH, 81477 München (DE)
(72) Inventor: KLINGER, Matthias, 81477 Munich (DE); ZUGMAIER, Gerhard, 81477 Munich (DE)
(74) Representative: Schiweck Weinzierl Koch Patentanwälte Partnerschaft mbB

(57) **Abstract**

The present invention in general relates to the field of medical treatments and diagnostic markers, and more specifically to methods and uses for treatment of acute lymphocytic leukemia (ALL) which involve administration of a B-cell depleting agent. Uses and methods for conserving or increasing the number of long term survivors of ALL are also envisaged. The uses and methods provided herein comprise administration of a B-cell depleting agent and/or adjusting of the treatment regimen or dosage of the B-cell depleting agent so that the number of B-cells in the blood of the treated patients remains or falls below one B-cell/ml serum within a predefined period of time.

## Description

### BACKGROUND

Acute lymphoblastic leukemia (ALL) is a progressive, malignant neoplasm of the blood-forming organs, typically characterized by large numbers of immature hematopoietic cells (particularly lymphoblasts) in the bone marrow, circulating blood, lymph nodes, spleen, liver, and other organs. During progression of the disease, lymphoblasts accumulate in the bone marrow, ultimately replacing most of the normal hematopoietic cells, resulting in anemia and increased susceptibility to infection and hemorrhage. Other typical symptoms include fever, pain in the joints and bones, and swelling of the lymph nodes, spleen, and liver. The total incidence of ALL is 1.1/100,000 per year. The incidence has its peak during childhood, decreasing continuously with increasing age. From the age of 35 years on the incidence rises again and a second peak is observed starting from the age of 80 years (2.3/100,000 per year) (Hoelzer and Gökbuget; Der Onkologe 12 (2006); 983-1002). Although the etiology of ALL still remains to be elucidated, it is one of the most carefully studied and best characterized neoplasms. ALL subgroups are defined mainly by immunophenotyping, cytogentics and molecular genetics. B-lineage acute lymphoblastic leukemia (ALL) with 74% of cases comprises the majority of ALL's. Seventy percent of all ALL's are B-precursor ALL's and 4% are mature B-cell ALL's. T-lineage ALL's covers 26% of all ALL's (Hoelzer and Gökbuget; Der Onkologe 12 (2006); 983-1002).

In the early 1980s, adult acute lymphoblastic leukemia (ALL) was a rarely curable disease with an overall survival of less than 10%. In the last years, progress has been made in molecular diagnostics of ALL. Stem cell transplantation (SCT) has improved the outcome of ALL and has made treatment more feasible. Though various new targeted drugs are under evaluation, effective targeted therapies for ALL are not yet available. An extensive review found that although children could obtain remission rates of up to 100%, disease-free survival at ten years was 63% for children and 25-35% for adults (Redaelli et al. Eur J Cancer Care (Engl). 2005 Mar;14(1):53-62). Rapid diagnosis and classification of ALL is increasingly important to identify prognostic and molecular genetic subsets that will be the focus of targeted treatment (Hoelzer and Gökbuget; Hematology (2006); 133-141).

ALL treatment typically today takes place in 3 phases: induction (or remission induction), consolidation (intensification) and maintenance and involves the long-term use of chemotherapy. The total treatment usually takes about 2 years, with the maintenance phase taking up most of this time. In the past several years, more intensive chemotherapy regimens have been used, which led to better responses to treatment. But these regimens are also more likely to cause side effects, such as low white blood cell counts, which in turn require administration of other drugs that help to prevent or treat these side effects. An important part of treatment for ALL is central nervous system (CNS) prophylaxis - treatment that is meant to ensure the leukemia does not spread to the brain or spinal cord.

Survival rates with modern treatment protocols for ALL patients have reached a plateau where the potential benefit of more aggressive chemotherapeutic regimens is often offset by an excess mortality due to complications, thus making efforts to individualize treatment even more important. This shows that acute lymphoblastic leukemia (ALL) remains for most patients a fulminate and incurable disease. In light of this, there is an urgent need for improved ALL therapies.

### SUMMARY

The present invention relates to methods and uses for treatment of ALL which involve administration of a B-cell depleting agent. Uses and methods for conserving or increasing the number of long term survivors of ALL are also envisaged. The uses and methods provided herein involve the number of B-cells in the blood of a patient (or group of patients) remaining or falling below one B-cell/ml serum within a predefined period of time.

In a first aspect, the present invention relates to a method of conserving or increasing the number of long term survivors in a group of patients suffering from ALL. The method comprises the step of administering a (therapeutically effective amount of) B cell depleting agent to said patients and/or adjusting the treatment regimen or dosage of a B cell depleting agent such that the number of B-cells in the blood of (preferably all of) the patients of said group remains or falls below one B cell/ml serum within a predefined period of time after the initial treatment with said B cell depleting agent.

In a second aspect a method of treating ALL in a patient or in a group of patients is provided, the method comprising administering a (therapeutically effective amount of) B cell depleting agent to said patients such that the number of B-cells in the blood of said patient or said group of patients remains or falls below one B cell/ml serum within a predefined period of time after the initial treatment with said B cell depleting agent.

In a third aspect, the present invention relates to a method of treating ALL in a patient or in a group of patients comprising the following steps:
(a) administering a B cell depleting agent to said patient or said group of patients,
and (b) determining or monitoring whether the number of B-cells in the blood of said patient or in said group of patients remains or falls below one B cell/ml serum within a first predefined period of time after the initial treatment with said B cell depleting agent.

Said method can further comprise the step of
(c) adjusting the B cell depleting agent such that the number of B-cells in the blood of said patient or said group of patients remains or falls below one B cell/ml serum within a predefined period of time after the initial treatment with said B cell depleting agent.

In a further aspect, a B cell depleting agent for use in a method of treating ALL is provided, said B cell depleting agent to be administered or adjusted such that the number of B-cells in the blood of the ALL patient remains or falls below one B cell/ml serum within a predefined period of time after the initial treatment with said B cell depleting agent.

In a further aspect, the present invention relates to a B cell depleting agent for use in a method of treating ALL, wherein said B cell depleting agent is to be administered or adjusted such that the number of B-cells in the blood of the ALL patient falls below one B cell/ml serum within a predefined period of time after the initial treatment with said B cell depleting agent.

In a further aspect, a B cell depleting agent for use in a method of treating ALL in a group of ALL patients is provided, said method comprising the steps of
(a) administering a B cell depleting agent,
and (b) determining or monitoring whether the number of B-cells in the blood of said patient remains or falls below one B cell/ml serum within a first predefined period of time after the initial treatment with said B cell depleting agent.

In a further aspect, the present invention relates to a B cell depleting agent for use in a method of conserving or increasing the number of long term survivors in a group of patients suffering from ALL.

Said method comprises the step of administering a B cell depleting agent and/or adjusting the treatment regimen or dosage of a B cell depleting agent such that the number of B-cells in the blood of the patients of said group falls below one B cell/ml serum within a (first) predefined period of time after the initial treatment with said B cell depleting agent.

In a further aspect, the present invention relates to the use of the number of B cells in the blood of an ALL patient for adjusting the dosage or treatment regimen with a B cell depleting agent such that the number of B-cells in the blood of said patient remains or falls below one B cell/ml serum within a (first) predefined period of time after the initial treatment with said B cell depleting agent.

In a further aspect, the present invention relates to the use of a blood sample of an ALL patient for determining the number of B cells for adjusting the dosage or treatment regimen with a B cell depleting agent such that the number of B-cells in the blood of said patient remains or falls below one B cell/ml serum within a predefined period of time after the initial treatment with said B cell depleting agent.

In a further aspect, a method of adjusting the dosage or treatment regimen with a B cell depleting agent in a patient suffering from ALL is provided.

Said method comprises the step of observing the number of B-cells in the blood of said ALL patient within a predefined first period of time after the initial treatment with said B cell depleting agent and adjusting the dosage or treatment regimen such that the number of B-cells in the blood of said patient remains or falls below one B cell/ml serum within a predefined period of time after the initial treatment with said B cell depleting agent.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

### DESCRIPTION OF THE FIGURES

**Figure 1****.** Relapse-free survival and overall survival (A) Relapse-free survival (RFS) in the entire patient population of the study (N = 36) at median follow-up of 28.9 months. (B) Overall survival with and without censoring for minimal residual disease in the entire patient population (N = 36) at a median follow-up of 32.6 months.
**Figure 2****.** Overall survival by prior allogeneic stem cell transplantation. Overall survival in the entire patient population of the study (N = 36) with and without prior allogeneic stem cell transplantation at a median follow-up of 32.6 months.
**Figure 3****.** T-cell and B-cell kinetics. T-cell and B-cell kinetics during cycle 1 (days 1 to 29) and cycle 2 (days 43 to 71) of blinatumomab treatment. (A) CD3⁺ T-cell expansion. (B) CD3⁺ T_{EM}-cell expansion. (C) CD19⁺ B-cell depletion kinetics. For clarity, initial T-cell redistribution during the first treatment week of cycles 1 and 2 is not shown. Patients were grouped according to overall survival. Mean cell values (±SEM) are plotted for each of the following groups: OS < 30 months; OS ≥ 30 months; OS ≥ 30 months without further treatment post blinatumomab. OS, overall survival.

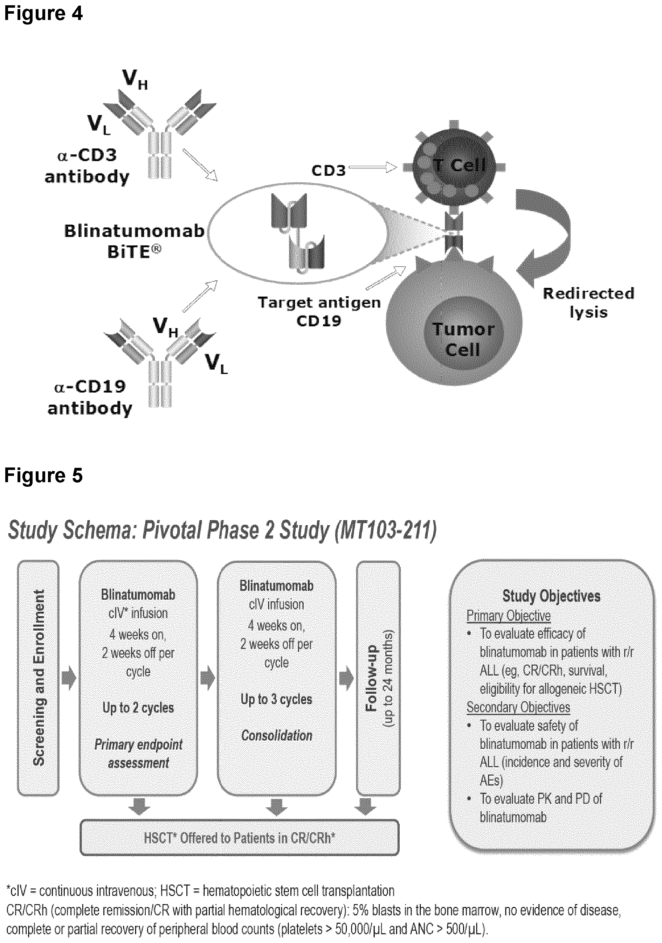

**Figure 4****.** Supposed mechanism of action of blinatumomab.
**Figure 5****.** Study scheme of blinatumomab phase 2 study.

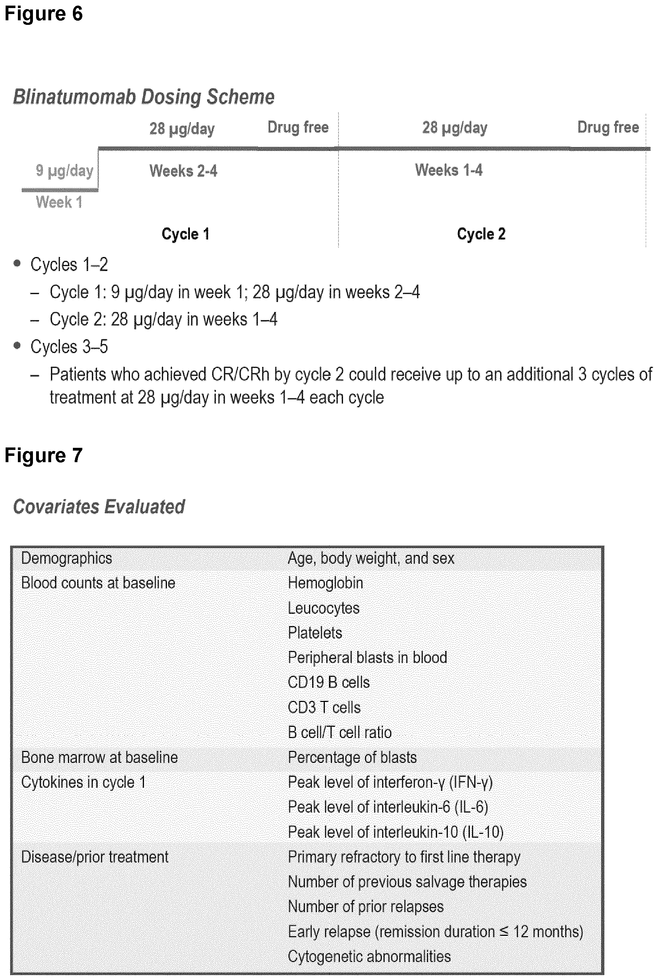

**Figure 6****.** Blinatumomab dosing scheme.
**Figure 7****.** Evaluated covariates.

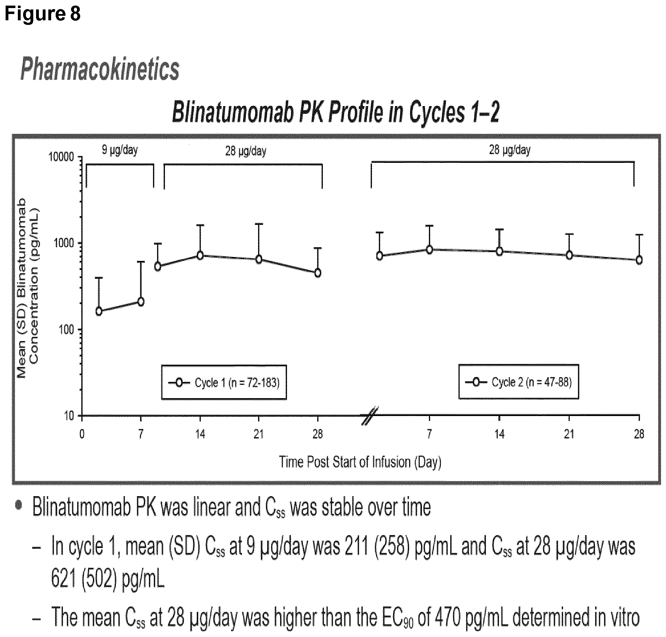

**Figure 8****.** Blinatumomab pharmacokinetics. Blinatumomab serum concentration was measured in all patients at baseline and at regular intervals during the first two treatment cycles. Blinatumomab concentrations were determined using a validated bioassy.

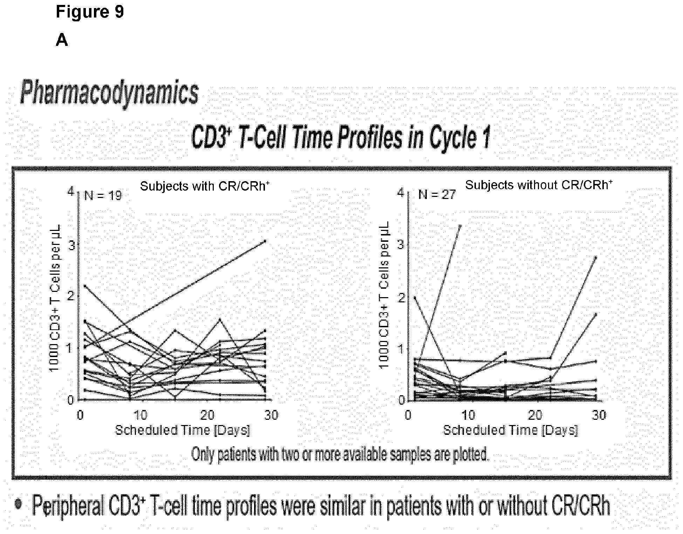

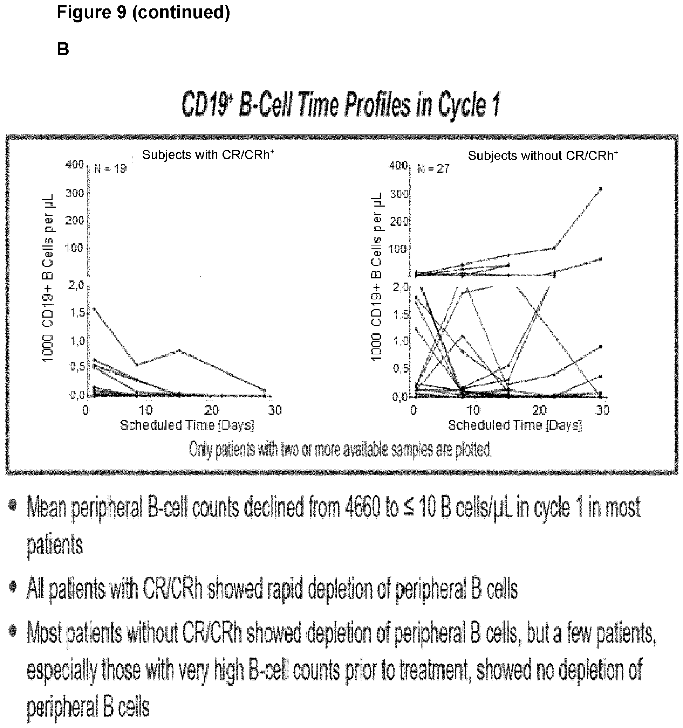

**Figure 9****.** Pharmacodynamics. Lymphocyte subsets (eg, CD19+ B cells, CD3+ T cells) were measured in i) a subgroup of patients (n = 46): samples collected at screening and during the first two treatment cycles ii) in the remaining patients: samples collected at screening and 6 months after treatment start. Lymphocyte subsets were measured by flow cytometry. A: CD3⁺ T cell time profiles in Cycle 1. B: CD19+ B cell time profiles in Cycle 1.

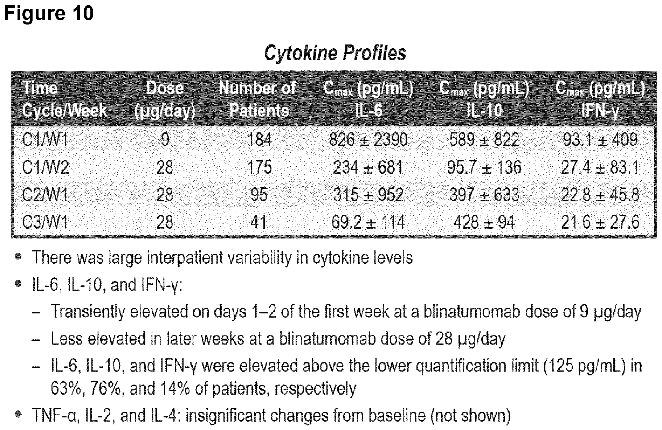

**Figure 10****.** Cytokine Profiles. Cytokine serum levels of TNF-α, IL-2, IL-4, IL-6, IL-10, and IFN-γ were determined. Cytokines were quantified with a validated FACS-based assay.

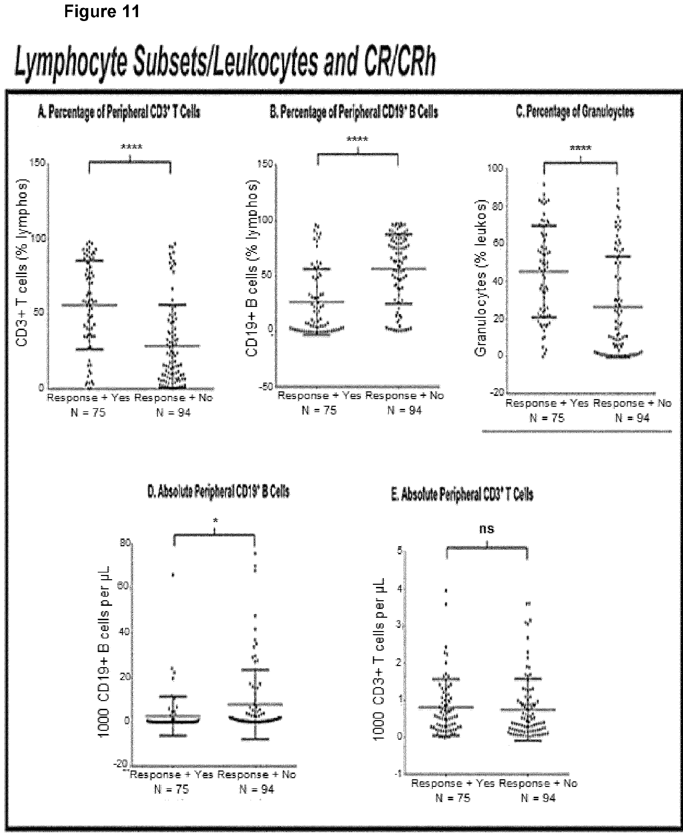

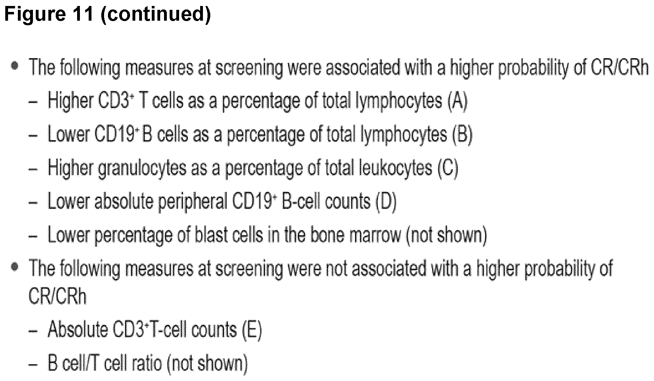

**Figure 11****.** Lymphocyte subsets/leucocytes and CR/CRh.

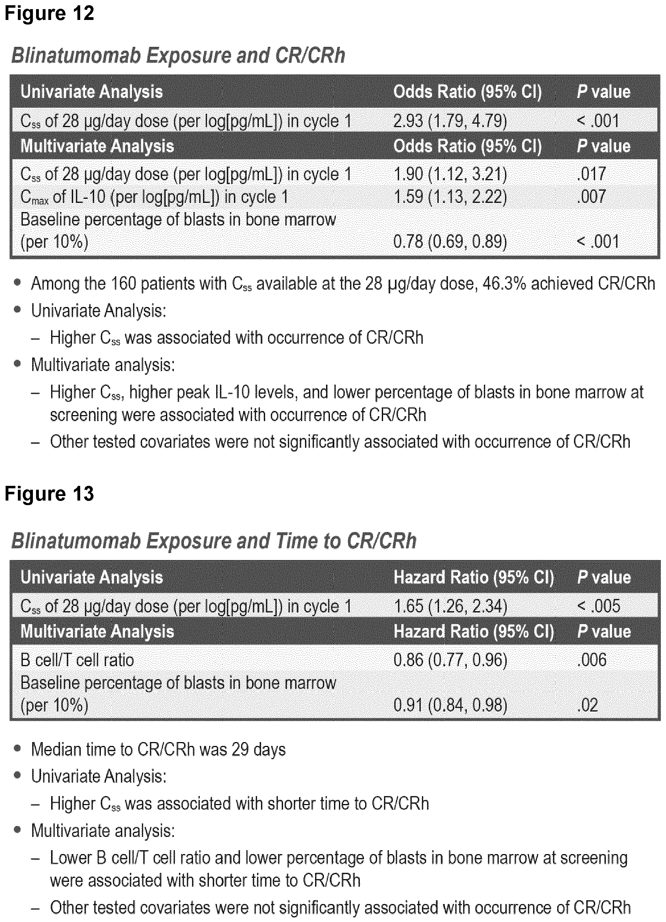

**Figure 12****.** Blinatumomab Exposure and CR/CRh
**Figure 13****.** Blinatumomab Exposure and Time to CD/CRh.

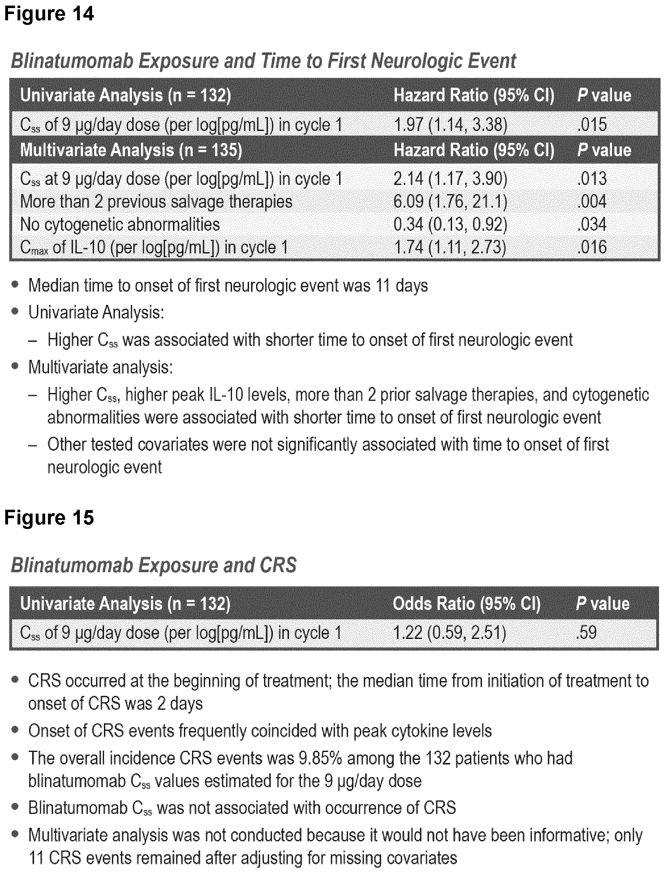

**Figure 14****.** Blinatumomab Exposure and Time to First Neurologic Event.
**Figure 15****.** Blinatumomab Exposure and Cytokine Release Syndrome (CRS).

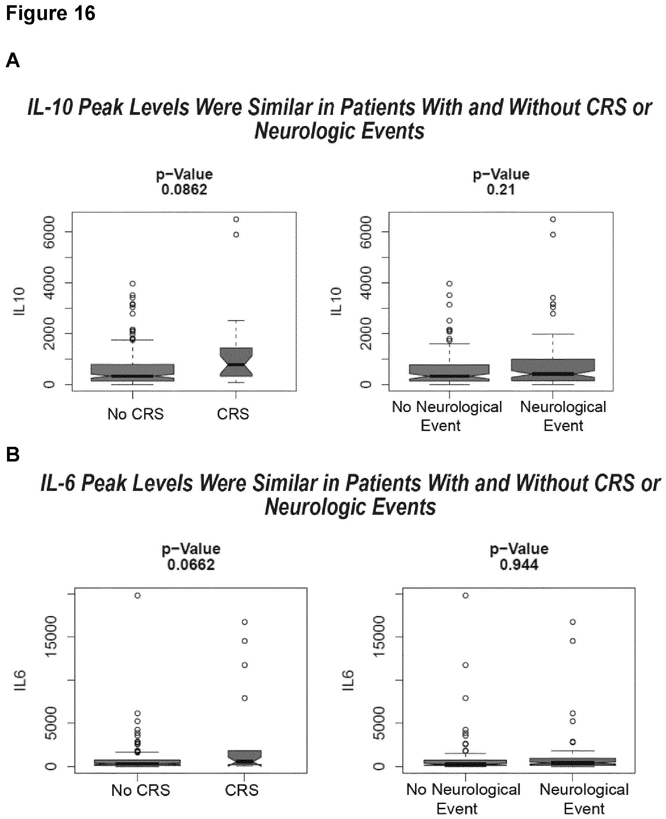

**Figure 16****.** IL10 and IL6 Peak Levels in patients with or without Cytokine Release Syndrome (CRS)..

### DETAILED DESCRIPTION

The present inventors have discovered a correlation between (peripheral) B-cell depletion within a defined period of time (8 days) and survival for ≥ about 29 months in ALL patients treated with the B-cell depleting agent blinatumomab. In contrast, the present inventors observed that in patients where B-cell depletion took 22 days and did not sustain, patients were likely to die in < about 29 months. The present inventors therefore concluded that when B-cell depletion can be successfully achieved in a patient (or group of patients) within a specific timeframe, the patient (or group of patients) is likely to be a long-term survivor of ALL and/or to respond favorably to subsequent ALL treatment. Without wishing to be bound by theory, it is speculated that the observed correlation could be attributed to an expansion of the T- and/or TEM-cell population in patients exhibiting a clearance of (peripheral) B cells which may further enhance subsequent ALL treatment via T-cell mediated anti-cancer activity. The means and methods provided herein therefore allow the skilled medical practitioner to quickly evaluate the likelihood of a beneficial outcome of treatment with a given B-cell depleting agent, and hence reduce the administration of agents for treatment of ALL to unresponsive patients. Hence, the present invention provides means and methods for a tailor-made ALL therapy that reduces the risk of side-effects, saves valuable time for treatment with suitable agents, and prevents unnecessary medicinal exposure.

The uses and methods described herein therefore include stratification of ALL patients. "Stratification" refers to sorting patients into those who may or may not benefit from subsequent ALL therapy, in particular ALL therapy involving administration of the B-cell depleting agent applied in the inventive methods and uses.

In accordance with the foregoing, the present invention provides a method of conserving or increasing the number of long term survivors in a group of patients suffering from ALL. In the aforementioned method, a (therapeutically effective) amount of B cell depleting agent is administered to said group of patients and/or the treatment regimen or dosage of a B cell depleting agent is adjusted such that the number of B-cells in the blood of said patients remains or falls below one B cell/ml serum within a predefined period of time after the initial treatment with said B cell depleting agent. Also provided herein is a B-cell depleting agent for use in the described method.

The above-mentioned method is envisaged to be useful in conserving or increasing the number of long-term survivors in a group of ALL patients. I.e., with the help of said method, numbers of long-term survivors in the group of patients treated according to the inventive method ("treated patients") will be preferably at least equal to the number of long-term survivors in the group of patients not treated according to the inventive method ("untreated patients"). Preferably, the number of long-term survivors in the group of treated patients will even exceed the number of long-term survivors in the group of untreated patients. Notably, "untreated patients" may include patients having received another ALL treatment, such as an ALL treatment as described in the section "further ALL treatment".

Further provided herein is a method of treating ALL in a patient or a group of patients. The method comprises administration of a (therapeutically effective) amount of B-cell depleting agent to said patients, such that the number of B-cells in the blood of said patient or said patients remains or falls below one B-cell/ml serum within a predefined period of time after the initial treatment with said B-cell depleting agent. A B-cell depleting agent for use in said method is also provided.

### ALL

"Acute lymphoblastic leukemia" or "ALL", also known as acute lymphocytic leukemia or acute lymphoid leukemia, generally refers to an acute form of leukemia which is typically characterized by the overproduction and/or accumulation of cancerous, immature white blood cells (also referred to as lymphoblasts). As used herein, the term "ALL" includes acute, refractory and relapsed ALL. The term "refractory ALL" as used herein means resistance of the ALL to conventional or standard ALL therapy, such as chemotherapy and/or hematopoietic stem cell transplantation (HSCT), i.e. the conventional or standard ALL therapy is not able to ultimately cure all ALL patients. The term "relapsed ALL" as used herein denotes the return of signs and symptoms of the ALL disease after a patient has enjoyed a remission. For example, after conventional ALL treatment using chemotherapy and/or HSCT, a ALL patient may go into remission with no sign or symptom of the ALL, remains in remission for a couple of years, but then suffers a relapse and has to be treated once again for ALL. The term "ALL" as used herein also includes minimal residual disease (MRD) in a patient with ALL, i.e. the presence of a small numbers of cancerous lymphoblasts remaining in the patient during treatment, or after treatment when the patient is in remission.

The term "ALL" generally encompasses B-cell ALL and T-cell ALL. The term "cancerous" is used herein interchangeably with the term "malignant" to designate cells that are not self-limited in their growth, are capable of invading into adjacent tissues, and may be capable of spreading to distant tissues (metastasizing).

It is envisaged that the methods and uses disclosed herein are particularly useful for treating B-cell ALL, including B-precursor ALL, such as pro-B ALL, pre-B ALL, or common ALL (cALL), and mature B-cell ALL (Burkitt leukemia). The term "ALL" includes both pediatric ALL and adult ALL. The means and methods of the present invention are in particular envisaged to be useful for treatment of relapsed and/or refractory adult B-precursor ALL.

The term "patient" includes all mammals, but is not limited to mouse, rat, dog, horse, camel, primates, etc., primates being preferred and humans, including children and adults, being most preferred. When used herein, the term "subject" is used interchangeably with the term "patient". What is disclosed with reference to a "patient" herein also applies to a group of patients, mutatis mutandis.

The term "pediatric ALL" or "pediatric ALL patient" as referred to herein denotes children aged from one month to 18 years. The indicated age is to be understood as the age of the children at diagnosis of the ALL disease. Both time intervals specifically include the upper limit and also the lower limit. This means that for example a time interval "from one month to 18 years" includes "one month" and "18 years". WO 2010/052013 provides means and methods for treating pediatric or childhood ALL, particularly refractory and/or relapsed pediatric ALL.

The term "adult ALL" or "adult ALL patient" as referred to herein denotes adults aged more than 18 years, i.e. patients aged 19, 20, 21, 22, 23, 24, 25, 30, 35, 40, or 50 years or more. Even patients with 70, 75, 80, 85, 90, 100 years or older may be treated by the methods and means of the invention. The indicated age is to be understood as the age of the adult at diagnosis of the ALL disease. WO 2010/052014 provides means and methods for treating adult ALL.

The term "long term survivor" is used herein to refer to patients living for 18 months or longer from initial treatment with the applied B-cell depleting agent, i.e. the time point of receiving the first dose of B-cell depleting agent, e.g. about 18 months, about 20 months, about 25 months, about 30 months, or more. It is particularly envisaged that the long term survivors live for about 30 months or more after receiving the first dose of b-dell depleting agent.

### B-cell depleting agent

The uses and methods of the present invention involve administration of a (therapeutically effective amount of) B cell depleting agent to a patient (or a group of patients).

In general, any route of administration is conceivable depending, e.g., on the formulation, bioavailability and mechanism of action of the B-cell depleting agent. E.g., the B-cell depleting agent can be administered orally, topically, transdermally, subcutaneously, intravenously, intraperitoneally, intramuscularly or intraocularly. However, any other route may readily be chosen by the person skilled in the art if desired. By "therapeutically effective amount" is meant an amount of the B-cell depleting agent that elicits a desired therapeutic effect, e.g. alleviation or amelioration (complete or partial) of the symptoms or condition of the ALL patient (or group of patients), or any other desired improvement in the patient's (or group of patients') symptoms, disease or condition. The exact amount dose may depend on, e.g., age, body weight, general health, sex, diet, drug interaction and the severity of the condition, as will be ascertainable with routine experimentation by those skilled in the art.

The term "B cell depleting agent" in general refers to an agent capable of reducing and/or controlling the number of B-cells in a patient.

The term thus includes agents that directly or indirectly destroy of some or all B-cells, e.g. by induction of cell death signals, antibody dependent cell-mediated cytotoxicity (ADCC), complement dependent cytotoxicity (CDC), or engagement of cytotoxic T-cells, and agents that block B cell activation or development. The term "B-cell" includes progenitor (or pre-pro) B cells, early pro (or pre-pre)-B cells, late pro (or pre-pre)-B cells, large pre-B cells, small pre-B cells, immature B cells and mature B cells. B cell depletion can be partial or complete, i.e. affect all B-cells or subpopulations of B-cells. Preferred B-cell depleting agents for use in the methods of the invention can reduce (or maintain) the level of B-cells in the blood of a patient (or group of patients) within a predefined period of time to one B-cell/ml serum or less as ascertainable by the skilled person using routine experimentation as described herein. It is in particular envisaged that B-cell depleting agents used in the methods of the invention are capable of depleting peripheral CD19+ B-cells.

B-cell depleting agents are known in the art and include, without limitation, co-stimulation blockers (abatacept and 7-related protein-1), cytokines (tocilizumab and baminercept), B cell receptor-targeted agents (abetimus and edratide), agents targeting CD20, CD22, CD19, CD40-CD40L, B cell activating factor belonging to the TNF family (BAFF) or A proliferation-inducing ligand (APRIL). In accordance with the foregoing, exemplary B-cell depleting agents useful in the methods of the invention include anti-CD20 agents (e.g., anti-CD20 antibodies such as rituximab, ofatumumab, ocrelizumab, veltuzumab, tositumomab, ibritumomab), anti-CD25 agents (e.g., anti-CD25 antibodies such as alemtuzumab), BAFF inhibitors (e.g., belimumab, atacicept), anti-CD154 agents (e.g. anti-CD154 antibodies such as ruplizumab, toralizumab), anti-CD19 agents (e.g., MDX-1342), anti-CD22 agents (e.g., epratuzumab) and anti-thymocyte globulin (ATG).

In particular, agents targeting CD19 and CD22 are envisaged, such as bispecific CD19xCD22 single chain antibodies, and particularly blinatumomab. Without wishing to be bound by theory, it is thought that blinatumomab transiently links CD19⁺ B cells to CD3⁺ T-cells, thereby inducing T-cell mediated serial lysis of B-cells and concomitant T-cell proliferation.

Blinatumomab comprises the
(a) anti-CD3 CDRs of the heavy chain shown as CD3 CDR-H1 in SEQ ID NO: 11 (GYTFTRYTMH), CD3 CDR-H2 in SEQ ID NO: 12 (YINPSRGYTNYNQKFKD) and CD3 CDR-H3 in SEQ ID NO: 13 (YYDDHYCLDY); and/or
(b) anti-CD3 CDRs of the light chain shown as CD3 CDR-L1 in SEQ ID NO: 14 (RASSSVSYMN), CD3 CDR-L2 in SEQ ID NO: 15 (DTSKVAS) and CD3 CDR-L3 in SEQ ID NO: 16 (QQWSSNPLT); and/or
(c) anti-CD19 CDRs of the heavy chain shown as CD19 CDR-H1 in SEQ ID NO: 17 (GYAFSSYWMN), CD19 CDR-H2 in SEQ ID NO: 18 (QIWPGDGDTNYNGKFKG) and CD19 CDR-H3 in SEQ ID NO: 19 (RETTTVGRYYYAMDY); and/or
(d) anti-CD19 CDRs of the light chain shown as CD19 CDR-L1 in SEQ ID NO: 20 (KASQSVDYDGDSYLN), CD19 CDR-L2 in SEQ ID NO: 21 (DASNLVS) and CD19 CDR-L3 in SEQ ID NO: 22 (QQSTEDPWT).

In the alternative, Blinatumomab comprises the
(a) CD19 variable heavy chain shown in SEQ ID NO: 3 (nucleotide sequence is shown in SEQ ID NO: 4); and/or
(b) CD19 variable light chain shown in SEQ ID NO: 5 (nucleotide sequence is shown in SEQ ID NO: 6); and/or
(c) CD3 variable heavy chain shown in SEQ ID NO: 7 (nucleotide sequence is shown in SEQ ID NO: 8); and/or
(d) CD3 variable light chain shown in SEQ ID NO: 9 (nucleotide sequence is shown in SEQ ID NO: 10).

In a further alternative, Blinatumomab comprises an amino acid sequence selected from the group consisting of
(a) an amino acid sequence as depicted in SEQ ID NO: 1;
(b) an amino acid sequence encoded by a nucleic acid sequence as shown in SEQ ID NO: 2;
(c) an amino acid sequence encoded by a nucleic acid sequence having at least 70%, 80%, 90%, 95% or 99% identity to a nucleic acid sequence of (b), wherein said amino acid sequence is capable of specifically binding to CD3 and CD19; and
(d) an amino acid sequence encoded by a nucleic acid sequence which is degenerate as a result of the genetic code to a nucleotide sequence of (b), wherein said amino acid sequence is capable of specifically binding to CD3 and CD19.

An alternative to Blinatumomab as B-cell depleting agent is a CD3 x CD19 antibody, wherein the CD3 binding molecule thereof preferably comprises a VL region comprising CDR-L1, CDR-L2 and CDR-L3 selected from:
(a) CDR-L1 as depicted in SEQ ID NO: 27 of WO 2008/119567, CDR-L2 as depicted in SEQ ID NO: 28 of WO 2008/119567 and CDR-L3 as depicted in SEQ ID NO: 29 of WO 2008/119567;
(b) CDR-L1 as depicted in SEQ ID NO: 117 of WO 2008/119567, CDR-L2 as depicted in SEQ ID NO: 118 of WO 2008/119567 and CDR-L3 as depicted in SEQ ID NO: 119 of WO 2008/119567; and
(c) CDR-L1 as depicted in SEQ ID NO: 153 of WO 2008/119567, CDR-L2 as depicted in SEQ ID NO: 154 of WO 2008/119567 and CDR-L3 as depicted in SEQ ID NO: 155 of WO 2008/119567; and/or
comprises a VH region comprising CDR-H 1, CDR-H2 and CDR-H3 selected from:
(a) CDR-H1 as depicted in SEQ ID NO: 12 of WO 2008/119567, CDR-H2 as depicted in SEQ ID NO: 13 of WO 2008/119567 and CDR-H3 as depicted in SEQ ID NO: 14 of WO 2008/119567;
(b) CDR-H1 as depicted in SEQ ID NO: 30 of WO 2008/119567, CDR-H2 as depicted in SEQ ID NO: 31 of WO 2008/119567 and CDR-H3 as depicted in SEQ ID NO: 32 of WO 2008/119567;
(c) CDR-H1 as depicted in SEQ ID NO: 48 of WO 2008/119567, CDR-H2 as depicted in SEQ ID NO: 49 of WO 2008/119567 and CDR-H3 as depicted in SEQ ID NO: 50 of WO 2008/119567;
(d) CDR-H1 as depicted in SEQ ID NO: 66 of WO 2008/119567, CDR-H2 as depicted in SEQ ID NO: 67 of WO 2008/119567 and CDR-H3 as depicted in SEQ ID NO: 68 of WO 2008/119567;
(e) CDR-H1 as depicted in SEQ ID NO: 84 of WO 2008/119567, CDR-H2 as depicted in SEQ ID NO: 85 of WO 2008/119567 and CDR-H3 as depicted in SEQ ID NO: 86 of WO 2008/119567;
(f) CDR-H1 as depicted in SEQ ID NO: 102 of WO 2008/119567, CDR-H2 as depicted in SEQ ID NO: 103 of WO 2008/119567 and CDR-H3 as depicted in SEQ ID NO: 104 of WO 2008/119567;
(g) CDR-H1 as depicted in SEQ ID NO: 120 of WO 2008/119567, CDR-H2 as depicted in SEQ ID NO: 121 of WO 2008/119567 and CDR-H3 as depicted in SEQ ID NO: 122 of WO 2008/119567;
(h) CDR-H1 as depicted in SEQ ID NO: 138 of WO 2008/119567, CDR-H2 as depicted in SEQ ID NO: 139 of WO 2008/119567 and CDR-H3 as depicted in SEQ ID NO: 140 of WO 2008/119567;
(i) CDR-H1 as depicted in SEQ ID NO: 156 of WO 2008/119567, CDR-H2 as depicted in SEQ ID NO: 157 of WO 2008/119567 and CDR-H3 as depicted in SEQ ID NO: 158 of WO 2008/119567; and
(j) CDR-H1 as depicted in SEQ ID NO: 174 of WO 2008/119567, CDR-H2 as depicted in SEQ ID NO: 175 of WO 2008/119567 and CDR-H3 as depicted in SEQ ID NO: 176 of WO 2008/119567.

The CD3 binding molecule of a CD3 x CD19 antibody may preferably comprise a VL region selected from the group consisting of a VL region as depicted in SEQ ID NO: 35, 39, 125, 129, 161 or 165 of WO 2008/119567; and/or comprise a VH region selected from the group consisting of a VH region as depicted in SEQ ID NO: 15, 19, 33, 37, 51, 55, 69, 73, 87, 91, 105, 109, 123, 127, 141, 145, 159, 163, 177 or 181 of WO 2008/119567.

The CD3 binding molecule of a CD3 x CD19 antibody may preferably comprise a VL region and a VH region selected from the group consisting of:
(a) a VL region as depicted in SEQ ID NO: 17 or 21 of WO 2008/119567 and a VH region as depicted in SEQ ID NO: 15 or 19 of WO 2008/119567;
(b) a VL region as depicted in SEQ ID NO: 35 or 39 of WO 2008/119567 and a VH region as depicted in SEQ ID NO: 33 or 37 of WO 2008/119567;
(c) a VL region as depicted in SEQ ID NO: 53 or 57 of WO 2008/119567 and a VH region as depicted in SEQ ID NO: 51 or 55 of WO 2008/119567;
(d) a VL region as depicted in SEQ ID NO: 71 or 75 of WO 2008/119567 and a VH region as depicted in SEQ ID NO: 69 or 73 of WO 2008/119567;
(e) a VL region as depicted in SEQ ID NO: 89 or 93 of WO 2008/119567 and a VH region as depicted in SEQ ID NO: 87 or 91 of WO 2008/119567;
(f) a VL region as depicted in SEQ ID NO: 107 or 111 of WO 2008/119567 and a VH region as depicted in SEQ ID NO: 105 or 109 of WO 2008/119567;
(g) a VL region as depicted in SEQ ID NO: 125 or 129 of WO 2008/119567 and a VH region as depicted in SEQ ID NO: 123 or 127 of WO 2008/119567;
(h) a VL region as depicted in SEQ ID NO: 143 or 147 of WO 2008/119567 and a VH region as depicted in SEQ ID NO: 141 or 145 of WO 2008/119567;
(i) a VL region as depicted in SEQ ID NO: 161 or 165 of WO 2008/119567 and a VH region as depicted in SEQ ID NO: 159 or 163 of WO 2008/119567; and
(j) a VL region as depicted in SEQ ID NO: 179 or 183 of WO 2008/119567 and a VH region as depicted in SEQ ID NO: 177 or 181 of WO 2008/119567.

The CD3 binding molecule of a CD3 x CD19 antibody may preferably comprise an amino acid sequence selected from the group consisting of SEQ ID NOs: 23, 25, 41, 43, 59, 61, 77, 79, 95, 97, 113, 115, 131, 133, 149, 151, 167, 169, 185 or 187 of WO 2008/119567.

The CD19 binding molecule of a CD19 x CD3 antibody is preferably characterized by the VH and/or VL regions or CDRs as described herein for Blinatumomab.

Whether a B-cell depleting agent is suitable for use in the inventive methods is readily ascertainable by the skilled person in the art using routine experimentation. E.g., the choice of a suitable B-cell depleting agent can depend on the type of ALL to be treated, and in particular the expression profile of the expanded B-cell population(s). For example, if the ALL to be treated is characterized by an expanding CD19⁺ B cell population, use of a B-cell depleting agent targeting CD19 is likely to be useful.

It is envisaged that the number of B-cells in the blood of the patient (or group of patients) remains or falls below one B cell/ml serum within the predefined period of time as defined herein.

In general, B-cell numbers can be evaluated through several techniques available in the art, e.g. using the white blood cell (WBC, or leukocytes) count and differential. White blood cells can be counted manually in hemocytometes (Neubauer chamber) or with automated counters. To determine the differential, a drop of blood can be thinly spread over a glass slide, air dried, and stained with a Romanofsky stain, most commonly the Wright or May-Grunewald-Giemsa technique. Cells are then counted and classified using morphologic examination and/or histochemistry as described in Blumenreich MS. The White Blood Cell and Differential Count. In: Walker HK, Hall WD, Hurst JW, editors. Clinical Methods: The History, Physical, and Laboratory Examinations. 3rd edition. Boston: Butterworths; 1990. Chapter 153. Alternatively, leukocytes are isolated from a blood sample and stained with fluorescent-labeled antibodies against lymphocyte cell surface markers and subsequently analyzed by flow cytometry as described in the appended examples. Percentages of each type of lymphocyte are multiplied with absolute lymphocyte numbers to calculate absolute cell numbers for each lymphocyte subpopulation.

It is envisaged that the number of B-cells in the blood of the treated patient (or treated group of patients) remains or falls below one B-cell/ml serum within a predefined period of time after the initial treatment with said B-cell depleting agent. The initial treatment with the applied B-cell depleting agent preferably means the first treatment with said applied B-cell depleting agent, i.e. the patient (or group of patients) has not received the applied B-cell depleting agent before. Said patient (or group of patients) may, however, have received further ALL treatments as described elsewhere herein before. It is also conceivable that the patient (or group of patients) has received another B-cell depleting agent before the initial treatment with the applied B-cell depleting agent. E.g., when a first B-cell depleting agent has no therapeutic effect, and/or fails to reduce (or maintain) the number of B-cells in the blood of a patient (or group of patients) to one B-cell/ml serum or less, a second B-cell depleting agent may be used. The initial treatment with the second B-cell depleting agent will then start on the first day of treating the patient (or group of patients) with the second B-cell depleting agent. That is, it is conceivable to apply the methods of the invention repeatedly (i.e., in several cycles) with different B-cell depleting agents, the "initial treatment" starting at the day of first treatment with the B-cell depleting agent of the respective cycle. The term "different" B-cell depleting agent also includes the same B-cell depleting agent as used in a preceding cycle, but in a different formulation, concentrations, or the like. It is also conceivable to repeat several cycles of treatment with the same B-cell depleting agent until the desired level of B-cells in the blood of the patient (or group of patients) is achieved.

The "predefined period of time" in which the number of B-cells remains or falls below one B-cell/ml serum is envisaged to be 15 days or less, i.e. 14, 13, 12, 11, 10, 9, 8, 7, 6, 5, 4, 3 days or less. The length of the predefined period of time is ascertainable by the skilled person in the art and may depend on the applied B-cell depleting agent, its concentration, treatment regimen, the type of ALL to be treated, and the like. Without wishing to be bound by theory, it is thought that when the number of B-cells can be adjusted to the desired number of one B-cell/ml serum or less within the predefined period of time, subsequent treatment is likely to be effective. A possible reason is the expansion of the T- and/or TEM-cell population in patients exhibiting a clearance of (peripheral) B cells, which may enhance the clinical activity of subsequent ALL therapies employing the cytotoxic potential of the T-cell system. "Subsequent treatment" may comprise further treatment with the B-cell depleting agent applied in the inventive method, with another B-cell depleting agent, further ALL treatment as described herein or combinations thereof after the B-cell numbers have been successfully reduced to (or maintained at) one B-cell per ml/serum or less within the predefined period of time. It is in general also conceivable that no subsequent treatment is necessary. In accordance with the foregoing, the methods of the invention therefore allow stratification of patients (or groups of patients), i.e. sorting patients (or groups of patients) into those who may or may benefit from subsequent ALL therapy. Also, it is envisaged that patients (or groups of patients) in which the number of B-cells in the blood can be reduced to or maintained at one B-cell/ml serum or less within the predefined period of time are likely to benefit from subsequent ALL therapy, while patients in which the number of B-cells in the blood exceeds one B-cell/ml serum within the predefined period of time, are not likely to benefit from subsequent ALL therapy.

The present invention also provides a method of treating ALL in a patient or in a group of patients comprising the following steps:
(a) administering a B cell depleting agent to said patient or said group of patients,
and (b) determining or monitoring whether the number of B-cells in the blood of said patient or in said group of patients remains or falls below one B cell/ml serum within a first predefined period of time after the initial treatment with said B cell depleting agent.

Said method can further comprise the step of
(c) adjusting the B cell depleting agent such that the number of B-cells in the blood of said patient or said group of patients remains or falls below one B cell/ml serum within a predefined period of time after the initial treatment with said B cell depleting agent. A B-cell depleting agent for use in the aforementioned methods is also provided.

In the aforementioned method, the number of B-cells in the blood of a patient (or group of patients) having received a B-cell depleting agent is monitored/determined after a first predefined period of time after the initial treatment with said B cell depleting agent. Means and methods for determining/monitoring the number of B-cells in the blood of a patient (or group of patients) have been defined elsewhere herein. Notably, the first period of time is shorter than the predefined period of time as defined elsewhere herein, and is preferably between 2 and 14 days, i.e. 2, 3, 4, 5, 6, 7, 8, 9, 10, 11, 12, 13, or 14 days. Step (b) of the inventive method is useful in determining whether or not the patient is responsive to the administration of said B-cell depleting agent, i.e. in stratifying patients in to those likely to benefit from subsequent ALL treatment after the first predefined period of time and those who are not. If the number of B-cells remains or falls below one B cell/ml serum within said first predefined period of time, then subsequent treatment is likely to be therapeutically effective.

If, however, the B-cell depleting agent e.g. fails to reduce or maintain the number of B-cells at the desired level of one B-cell/ml serum or less within the first predefined period of time, then the B-cell depleting agent can be adjusted such that the number of B-cells in the blood of the patient (or group of patients) remains or falls below one B cell/ml serum within a predefined period of time of preferably 15 days or less after the initial treatment with said B cell depleting agent.

In general, the first predefined period of time and the predefined period of time can be of any length, as long as the first predefined period of time is shorter than the predefined period of time. Both periods of time start with the first day of initial treatment with the B-cell depleting agent applied in the inventive methods, i.e. on the same day. The skilled practitioner will readily be able to determine the desired length of the first predefined period of time, depending on, e.g., the B-cell depleting agent, its concentration, formulation, treatment regiment, the ALL type, the physical constitution and findings of the patient (or group of patients), and the predefined period of time.

### Adjustment

Methods of the invention may further involve "adjusting" the B-cell depleting agent. The term "adjusting" refers to an intentional modification of the B-cell depleting agent in order to achieve a number one B-cell/ml serum or less in the treated patient (or group of patients). Thus, the number of B-cells in the blood of an ALL patient is used to adjust the dosage or treatment regimen with the B-cell depleting agent such that the number of B-cells in the blood of said patient remains or falls below one B cell/ml serum within a (first) predefined period of time after the initial treatment with said B cell depleting agent. The adjustment (intentional modification) may involve modification of the dosage, treatment regimen, formulation, or the like. For example, if the number of B-cells in the blood of a patient exceeds one B cell/ml serum within a (first) predefined period, a higher dosage of B-cell depleting agent may be administered, or the B-cell depleting agent may be administered for a prolonged period of time.

The exact dosage of B-cell depleting will depend on the purpose of the treatment (e.g. remission maintenance vs. acute flare of disease), route of administration, age, body weight, general health, sex, diet, time of administration, drug interaction and the severity of the condition, and will be ascertainable by one skilled in the art using known techniques.

Generally, any modification is conceivable as long as it preferably results in the number of B-cells in the blood of the treated patient (group of patients) being reduced to or maintained at a number of one B-cell/ml serum or less within the predefined period of time. The modification may also include additionally applying further ALL treatments as described herein, and/or administering another B-cell depleting agent.

In accordance with the foregoing, a method of adjusting the dosage or treatment regimen with a B cell depleting agent in a patient suffering from ALL is provided. Said method comprises the step of observing the number of B-cells in the blood of said ALL patient within a predefined first period of time after the initial treatment with said B cell depleting agent and adjusting the dosage or treatment regimen such that the number of B-cells in the blood of said patient remains or falls below one B cell/ml serum within a predefined period of time after the initial treatment with said B cell depleting agent.

### Further ALL treatment

It is also conceivable to use further ALL treatment in combination with the uses and methods of the invention. Further ALL treatment can in general be applied antecedently, simultaneously, and/or subsequently to the uses and methods of the invention.

Hematopoietic stem cell transplantation (HSCT) is a common ALL treatment. The term generally refers to transplantation of hematopoietic stem cells, usually derived from bone marrow or blood, and comprises autologous (i.e., the stem cells are derived from the patient) and allogeneic (i.e., the stem cells are derived from a donor) HSCT. For ALL treatment, allogeneic HSCT is generally preferred. It is also envisaged that the uses and methods of the present invention can be applied before or after HSCT, or both, or in between two HSCT treatments.

Patients (or groups of patients) treated according to the methods of the invention may also receive a chemotherapeutic treatment. In the context of the present invention, a "chemotherapeutic treatment" refers to a treatment with an antineoplastic agent or the combination of more than one of these agents into a standardized treatment regimen. In the context of the present invention, the term "chemotherapeutic treatment" comprises any antineoplastic agent including small sized organic molecules, peptides, oligonucleotides and the like. Agents included in the definition of chemotherapy are, without limitation, alkylating agents, e.g. mechlorethamine, cyclophosphamide, melphalan, chlorambucil, ifosfamide, busulfan, N-Nitroso-N-methylurea (MNU), carmustine (BCNU), lomustine (CCNU), semustine (MeCCNU), fotemustine, streptozotocin, dacarbazine, mitozolomide, temozolomide, thiotepa, mytomycin, diaziquone (AZQ), cisplatin, carboplatin, oxaliplatin, procarbazine and hexamethylmelamine; antimetabolites, e.g. methotrexate, pemetrexed, fluorouracil, capecitabine, cytarabine, gemcitabine, decitabine, Vidaza, fludarabine, nelarabine, cladribine, clofarabine, pentostatin, thioguanine, mercaptopurine; anti-microtubule agents e.g. vincristine, vinblastine, vinorelbine, vindesine, vinflunine, paclitaxel, docetaxel, podophyllotoxin; topoisomerase inhibitors, e.g. irinotecan, topotecan, etoposide, doxorubicin, mitoxantrone, teniposide, novobiocin, merbarone, aclarubicin; cytotoxic antibiotics, e.g. actinomycin, bleomycin, plicamycin, mitomycin, doxorubicin, daunorubicin, epirubicin, idarubicin, pirarubicin, aclarubicin, and mitoxantrone.

Further ALL treatment also includes radiation therapy. CNS treatment or prophylaxis is also envisaged in order to prevent malignant cells from spreading in the CNS, e.g. by intrathecal chemotherapy and/or radiation therapy of the brain and spinal cord.

Since the present inventors speculate that therapeutic success of ALL treatment could be based (in part) of an expansion of the T cell populations resulting in enhanced T-cell anti-cancer activity, inducers and enhancers of T cell activation and/or proliferation, CAR T cells, donor T cells, anti-cytotoxic T-lymphocyte-associated antigen-4 (CTLA-4) antibodies and others are also envisaged.

### Treatment

The term "treatment" in all its grammatical forms includes therapeutic or prophylactic treatment of ALL. A "therapeutic or prophylactic treatment" comprises prophylactic treatments aimed at the complete prevention of clinical and/or pathological manifestations or therapeutic treatment aimed at amelioration or remission of clinical and/or pathological manifestations. The term "treatment" thus also includes the amelioration or prevention of ALL. The term "treatment" as used herein means in the broadest sense medical procedures or applications that are intended to relieve illness. In the present case, the administration of a B-cell depleting agent (prepared for administration to an ALL patient) as described herein is for the treatment, amelioration or elimination of ALL in patients.

### Pharmaceutical composition

It is envisaged to administer the B-cell depleting agent in the form of a pharmaceutical composition. The term "pharmaceutical composition" particularly refers to a composition suitable for administering to a human or animal, i.e., a composition containing components which are pharmaceutically acceptable. Preferably, a pharmaceutical composition comprises a B-cell depleting agent together with one or more pharmaceutical excipients. The composition may also comprise further ALL agents as described elsewhere herein. The term "excipient" includes fillers, binders, disintegrants, coatings, sorbents, antiadherents, glidants, preservatives, antioxidants, flavoring, coloring, sweeting agents, solvents, co-solvents, buffering agents, chelating agents, viscosity imparting agents, surface active agents, diluents, humectants, carriers, diluents, preservatives, emulsifiers, stabilizers or tonicity modifiers. Pharmaceutical compositions of the invention preferably comprise a therapeutically effective amount of B-cell depleting agent and can be formulated in various forms, e.g. in solid, liquid, gaseous or lyophilized form and may be, inter alia, in the form of an ointment, a cream, transdermal patches, a gel, powder, a tablet, solution, an aerosol, granules, pills, suspensions, emulsions, capsules, syrups, liquids, elixirs, extracts, tincture or fluid extracts or in a form which is particularly suitable for the desired method of administration.

A better understanding of the present invention and of its advantages will be obtained from the following example, offered for illustrative purposes only. The example is not intended to limit the scope of the present invention in any way.

### EXAMPLE

### SUMMARY

A phase 2 study was conducted to evaluate the activity and safety of the bispecific T-cell engager (BiTE^{®}) antibody construct blinatumomab in 36 adults with relapsed/refractory B-precursor acute lymphoblastic leukemia (ALL). In the primary analysis, complete remission with full (CR) or partial (CRh) recovery of peripheral counts was observed in 25 (69%) patients and a minimal residual disease (MRD) response (< 10⁻⁴) in 25 (69%) patients (23 responders and 3 patients with hypocellular bone marrow). In this long-term follow-up analysis, median relapse-free survival was 8.8 months (median follow-up, 28.9 months), and median overall survival (OS) was 13.0 months (median follow-up, 32.6 months). An MRD response to blinatumomab was associated with significantly longer OS (p = 0.009). Ten patients (28%) were long-term survivors, defined as OS ≥ 30 months. Six long-term survivors received allogeneic stem cell transplantation (SCT) as consolidation for blinatumomab. Four long-term survivors did not receive allogeneic SCT as consolidation for blinatumomab, including three with no treatment after blinatumomab. One had CD19-negative relapse during the third cycle and achieved another CR with chemotherapy and allogeneic SCT as consolidation for chemotherapy. Neurologic events or cytokine release syndrome led to temporary blinatumomab discontinuation in three long-term survivors, all of whom restarted blinatumomab successfully. Long-term survivors had more rapid clearance of peripheral B-cells and significantly greater T-cell expansion than patients with OS < 30 months. These data suggest that long-term survival after blinatumomab treatment might be associated with a high degree of T-cell expansion and MRD response.

### INTRODUCTION

The prognosis is poor for adult patients with relapsed/refractory (r/r) B-precursor ALL. Treatment with chemotherapy has been reported to result in median overall survival (OS) from 4.5 to 8.4 months,¹⁻⁵ and 5-year OS rates are only 7% to 10%.^{1,2} Median OS is 5.8 months among patients who relapse after allogeneic stem cell transplantation (SCT), and 10 months among patients who relapse after chemotherapy only (without prior allogeneic SCT).⁵

Blinatumomab, a CD19/CD3 bispecific T-cell engager (BiTE^{®}) antibody construct, leads to redirected lysis of CD19-positive (CD19⁺) target B cells by inducing a transient cytolytic synapse between the target cells and T cells.⁶ In an exploratory dose-finding phase 2 study in adult patients with r/r B-precursor ALL (including patients in late first relapse >12 months), 69% of patients achieved a complete response (CR) or complete response with partial hematologic recovery (CRh), and 88% of responders achieved a minimal residual disease (MRD) response.⁷ In addition, MRD response was seen in three patients with hypocellular bone marrow. The study explored both fixed dosing as well as single-step and double-step dosing to prevent severe cytokine release syndrome (CRS). In a confirmatory phase 2 study of 189 patients with r/r B-precursor ALL, including those with early relapse (less than 12 months) after first remission, 43% achieved CR or CRh after two cycles of treatment with blinatumomab. Median relapse-free survival (RFS) was 5.9 months; median OS was 6.1 months.⁸

The first analysis of the aforementioned phase 2 dose-finding study had analyzed OS with a median follow-up of 12.1 months.⁷ The long-term follow-up analysis presented here evaluated OS at a median follow-up of 32.6 months. Long-term survivors were defined as patients who were alive for ≥ 30 months after starting blinatumomab treatment. We evaluated clinical characteristics, including disease-related medical history of the long-term survivors before blinatumomab treatment, outcomes of blinatumomab treatment, including hematologic and MRD responses to blinatumomab, adverse events, consolidation with allogeneic SCT, relapses, and T-cell as well as B-cell kinetics during treatment.

### PATIENTS AND METHODS

### Study Design

This report describes a follow-up analysis of relapse and overall survival; the methods of the primary analysis were described elsewhere.⁷ This was an open-label, multicenter, exploratory, single-arm, phase 2 study in adult patients with r/r B-precursor ALL conducted in collaboration with the German Study Group for Adult Acute Lymphoblastic Leukemia. The target population was Philadelphia chromosome (Ph)-negative and Ph-positive patients with primary refractory disease or relapse. Key exclusion criteria were Ph-positive ALL eligible for dasatinib or imatinib treatment; autologous SCT within 6 weeks and allogeneic SCT within 3 months before the start of blinatumomab treatment; history or presence of clinically relevant central nervous system (CNS) pathology, active CNS leukemia, active graft-versus-host disease (GVHD) and/or immunosuppressive therapy for GVHD within 1 week of blinatumomab treatment start, or active infections.⁷ The study protocol was approved by the Paul-Ehrlich-Institute and each study site's independent ethics committee, and written informed consent was obtained from all patients. Toxicity and efficacy data were reviewed by an independent data monitoring committee. ClinicalTrials.gov Identifier: NCT01209286.

### Study Procedures

The first 2 cycles of blinatumomab were administered to induce remissions. Bone marrow aspirate or biopsy was obtained before the first blinatumomab cycle and on day 29 of each cycle; cytomorphology and MRD were assessed in central reference laboratories. Hematologic complete remission with full recovery of peripheral blood counts (CR) was defined by ≤ 5% blasts in the bone marrow, no evidence of circulating blasts or extra medullary disease, platelets > 100,000/µL, hemoglobin ≥ 11 g/dL, and absolute neutrophil count (ANC) > 1,500/µL. Hematologic complete remission with partial recovery of peripheral blood counts (CRh) was defined by the same criteria but a lower minimum of peripheral blood counts (platelets > 50,000/µL, hemoglobin ≥ 7 g/dL, and ANC > 500/µL). An MRD response was defined as MRD < 10⁻⁴ by allele-specific real-time quantitative polymerase chain reaction for clonally rearranged immunoglobulin and/or T-cell receptor genes (sensitivity ≥ 10⁻⁴).⁹

Each treatment cycle was 6 weeks, including 4 weeks of continuous intravenous infusion and a 2-week treatment-free interval. The dose-finding stage used the following dosing schedules: Cohort 1 (n = 7) received blinatumomab 15 µg/m²/day; Cohort 2a (n = 5) received 5 µg/m²/day in week 1 and then 15 µg/m²/day; Cohort 2b (n = 6) received blinatumomab 5 µg/m²/day in week 1, 15 µg/m²/day in week 2, and then 30 µg/m²/day. In the extension stage, Cohort 3 (n = 18) used the dosing schedule from Cohort 2a. In case of CR or CRh, consolidation treatment with up to 3 additional cycles of blinatumomab and/or allogeneic SCT was permitted. After one incidence of grade 4 CRS, prephase treatment with dexamethasone (≤ 24 mg for 1-5 days) and/or cyclophosphamide (200 mg/m² for 1-4 days) was allowed. All patients had mandatory intrathecal CNS prophylaxis with methotrexate 15 mg, cytarabine 40 mg, and dexamethasone 4 mg administered by a spinal tap during screening and on day 29 of each cycle. Intravenous dexamethasone 16 mg or equivalent was given within 1 hour of treatment start. Adverse events were collected throughout the study and graded by the Common Terminology Criteria for Adverse Events (Version 4.0).¹⁰

### Analysis of Lymphocyte Subpopulations

Using methods that were described previously,¹¹ peripheral blood mononuclear cells were isolated at various time points before and throughout the first and second cycle of blinatumomab treatment and stained with fluorescent-labeled antibodies against the following cell surface markers: CD3⁺/CD13⁻/CD14⁻ or CD3⁺/CD45⁺ (T cells); CD3⁺/CD45RA⁻/CD197⁻ (effector memory T [T_{EM}] cells); and CD19⁺/CD13⁻/CD14⁻ or CD19⁺/CD45⁺ (B cells). Flow cytometry data was collected on a FACSCanto II (Becton Dickinson, Heidelberg, Germany), or Navios 10/3 instrument (Beckman Coulter, Krefeld, Germany). Statistics were analyzed by the software FCS Express (De Novo Software, Glendale, CA, USA), or Kaluza (Beckman Coulter). Percentages of lymphocyte subpopulations were multiplied by absolute lymphocyte numbers from a differential blood count to calculate absolute cell numbers for each lymphocyte subpopulation. Patients with complete datasets (T and T_{EM} cells, before [=baseline] and on days 8, 15, 22, and 29 of each cycle; B cells, additionally on day 3 of each cycle) were included in the analysis regardless of blinatumomab dosing regimen.

### Statistical Analysis

Relapse-free survival was measured from the time of first CR or CRh to hematologic or extra-medullary relapse or death resulting from any cause. Patients still in remission at data lock were censored at the time of last remission status assessment. Overall survival was measured from the time of first blinatumomab dose to death resulting from any cause. Kaplan-Meier methods were used to estimate the probability of RFS and OS over time, providing median and 95% confidence interval (CI). A Mantel-Byar test was conducted to evaluate the OS benefit associated with achieving an MRD response versus not achieving an MRD response. A log-rank test was conducted to compare OS between patients with prior allogeneic SCT versus those without prior allogeneic SCT. Long-term survivors were defined as patients with OS ≥ 30 months. Summary statistics were provided for long-term survivors, including clinical characteristics before blinatumomab treatment, use of allogeneic SCT before/after blinatumomab, treatment responses and relapses, and adverse events. Patients were grouped according to OS < 30 months, OS ≥ 30 months with further treatment, and OS ≥ 30 months without further treatment. The definition of long-term overall survival by duration of at least 30 months is based on published data, which show most events occurring with the first 24 months.⁵

### RESULTS

### Treatment Response and Survival

Thirty-six patients were treated at nine centers in Germany between October 6, 2010, and June 19, 2012, with follow-up ongoing. As described previously for the primary analysis,⁷ the rate of CR/CRh was 69% (25 of 36 patients). Fifteen patients (42%) achieved CR, and 10 patients (28%) achieved CRh. The remaining patients had partial remission (n = 2) or hypocellular bone marrow (n = 3); or were refractory to treatment (n = 4) or unevaluable (n = 2). Twenty-two of 25 patients with CR/CRh (88%) had an MRD response (three responders did not achieve an MRD response). An additional three patients with bone marrow that did not fulfill the criteria for partial hematologic recovery had an MRD response. Thus, 25 of 36 patients (69%) who were treated with blinatumomab had an MRD response.

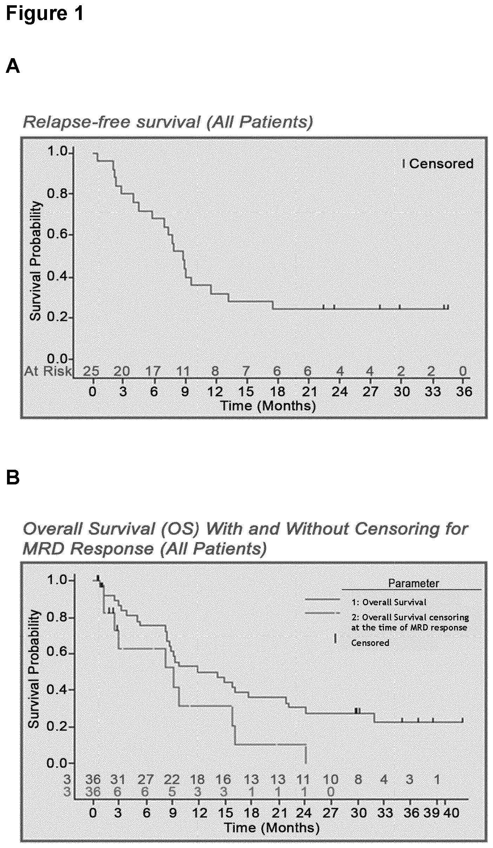

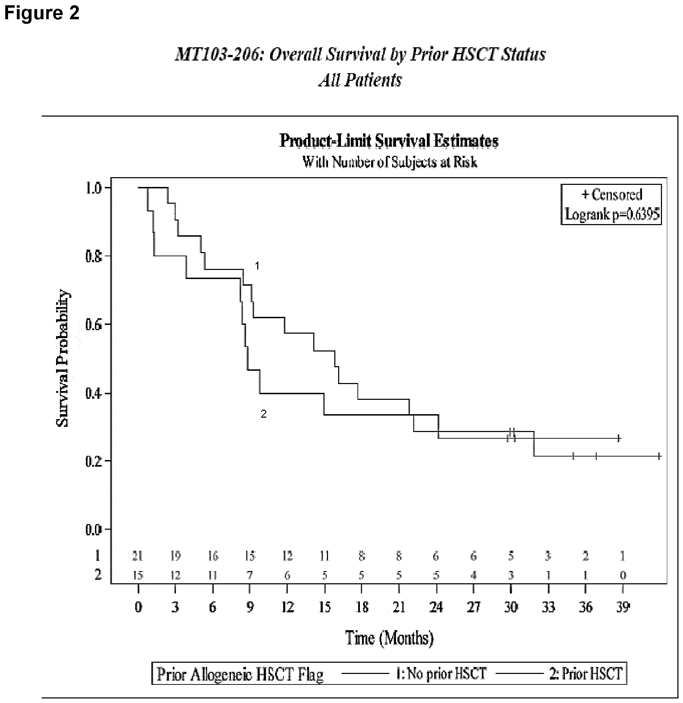

At a median follow-up time of 28.9 months, median RFS was 8.8 months (95% Cl, 5.7-13.2 months) (Figure 1A). At a median follow-up time of 32.6 months, median OS was 13.0 months (95% Cl, 8.5-21.9 months) (Figure 1B). A plateau was reached for RFS after approximately 18 months and for OS after approximately 33 months. When patients were censored for an MRD response in the analysis of OS, median OS was 9.0 months (95% Cl, 2.4-15.8 months); the Mantel-Byar odds ratio was 0.33 (P = .009), suggesting a 67% risk reduction associated with an MRD response (Figure 1B). There was no difference in OS between patients with prior allogeneic SCT and patients without prior allogeneic SCT (log-rank P = .6395; Figure 2).

### Clinical Characteristics of Long-term Survivors

Ten of 36 patients (28%) were long-term survivors, defined as OS ≥ 30 months after start of blinatumomab treatment. The main clinical characteristics of the long-term survivors are summarized in Table 1. A detailed listing of clinical characteristics for each individual patient is provided in Supplemental Table 1. Of the 10 patients, four had undergone allogeneic SCT before blinatumomab treatment and six had not. At screening, bone marrow blasts ranged from 8% to 97% and age ranged from 21 to 72 years. One of the 10 patients had Philadelphia chromosome-positive ALL.

Treatment responses, follow-up treatment, and relapses are summarized in Table 2. Most of the long-term survivors had achieved a CR (70%) but patients with CRh (20%) or hypocellular bone marrow (10%) were also among the long-term survivors. All long-term survivors had achieved an MRD response with blinatumomab treatment. Thus, 10 of 25 patients (40%) with an MRD response (including three with hypocellular bone marrow and MRD response) were long-term survivors, while none of the 11 patients without an MRD response was a long-term survivor.

Three of the long-term survivors relapsed after blinatumomab treatment, including two with CD19-positive relapse and one with CD19-negative relapse. One of the patients who relapsed received retreatment with blinatumomab, and two received chemotherapy. One patient relapsed within 12 months after start of blinatumomab treatment; two patients relapsed later than 12 months after start of blinatumomab treatment. Details for the patients who relapsed after blinatumomab treatment are provided in the following sections.

### Long-term Survivors With Allogeneic SCT as Consolidation for Blinatumomab

Six of the 10 long-term survivors underwent allogeneic SCT as consolidation for blinatumomab (Table 2). Three of six patients previously received allogeneic SCT before blinatumomab treatment. Five of six patients were still alive at the last follow-up for this analysis, including the three patients who received allogeneic SCT before blinatumomab. One of the three patients had achieved hypocellular bone marrow as treatment response to blinatumomab; the other patients achieved a CR or CRh as the best response to blinatumomab treatment.

One long-term survivor died of GVHD 31.9 months after the start of blinatumomab treatment. This patient received allogeneic SCT as consolidation for blinatumomab and had a CD19-positive relapse 10 months after allogeneic SCT. The patient achieved another remission with FLAG-IDA chemotherapy and received a second allogeneic SCT as consolidation for the chemotherapy.

### Long-term Survivors Without Allogeneic SCT as Consolidation for Blinatumomab

Four of the 10 long-term survivors did not undergo allogeneic SCT as consolidation for blinatumomab. All four achieved a CR or CRh as the best response to blinatumomab treatment. One of these four patients had received two prior allogeneic SCT. All four patients were still alive at the last follow-up for this analysis. Two of these four patients are in ongoing remission without further treatment. One patient has been alive for 3.5 years since the start of blinatumomab treatment (the patient had a reversible grade 4 CRS). The other patient has been alive for 4 years since the start of blinatumomab treatment (the patient had a reversible grade 3 neurological event). The other two patients relapsed after blinatumomab treatment. One of these patients had two CD19-positive relapses more than 12 months after having received 5 cycles of blinatumomab but responded to blinatumomab after each relapse with an MRD-negative remission (3 cycles of retreatment each time). The second patient had a CD19-negative relapse during the third cycle of blinatumomab. This patient achieved another remission after FLAG-IDA chemotherapy and received allogeneic SCT as consolidation for the chemotherapy. The patient was still alive at a follow-up of 46 months. Relapses in the central nervous system were not reported.

### Adverse Events in Long-term Survivors

Eight of the 10 long-term survivors had neurologic events, primarily headache and tremor (mostly grade 1), and two had CRS (grade 2 and 4, respectively). In the exploratory dose-finding study, three patients (8%) had CRS; and eight patients required treatment interruption because of neurologic events or CRS.⁷ In three of the long-term survivors grade 3 or 4 neurologic events or CRS resulted in treatment interruption (Table 3). All three patients achieved a CR or CRh as the best response to blinatumomab treatment and received no other treatment than blinatumomab (two of the three patients are still in remission). The first patient had grade 2 focal seizure in the first treatment cycle at a dose of 15 µg/m²/day. This patient resumed treatment, with antiseizure prophylaxis, at a dose of 5 µg/m²/day without further neurologic adverse events. The second patient had grade 3 encephalopathy at a dose of 30 µg/m²/day. This patient also resumed treatment at a dose of 5 µg/m²/day without further neurologic adverse events. The third patient had grade 4 CRS at a dose of 5 µg/m²/day. The patient had a marrow blast count of 90% before treatment and did not receive prephase treatment with dexamethasone or cyclophosphamide. The patient resumed treatment, with dexamethasone, at a blinatumomab dose of 5 µg/m²/day without further occurrence of CRS. When the dose was escalated to 15 µg/m²/day at the end of week 1, grade 2 CRS was recorded which was also mitigated by dexamethasone.

### T-Cell and B-Cell Kinetics

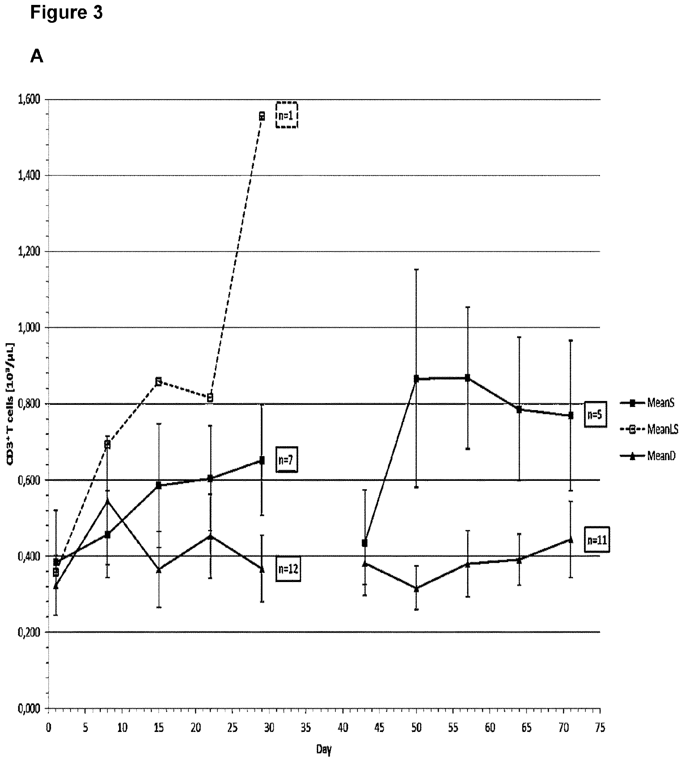

Expansion of CD3⁺ T cells, which was analyzed by kinetics of mean cell counts during treatment cycles 1 and 2, was predominantly observed in patients with OS ≥ 30 months, both in cycle 1 and in cycle 2 (Figure 3A). Of note, the most pronounced CD3⁺ T-cell expansion was observed in the long-term survivor who had a grade 3 neurologic adverse event and who had received no other treatment than 5 cycles of blinatumomab (Figure 3A). In spite of the extensive T-cell expansion, this patient did not show any symptoms or sign of GVHD. No T-cell kinetics were available for the long-term survivor with grade 4 CRS who also received only blinatumomab as treatment.

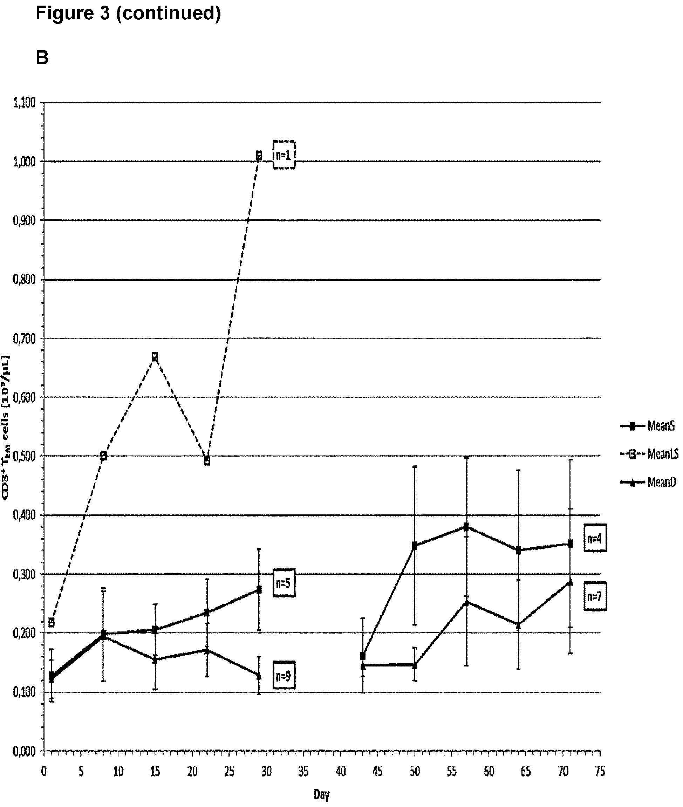

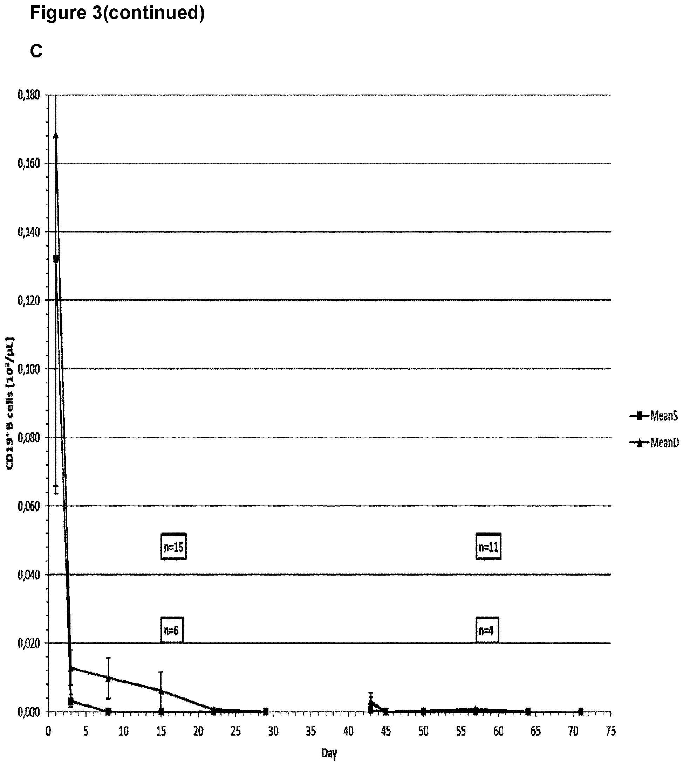

CD3⁺ T-cell expansion correlated with increasing numbers of CD3⁺ T_{EM} cells, which are known to be cytolytic and which play an important role in blinatumomab-mediated cytotoxicity against target B cells (Figure 3B). Again, the patient with the grade 3 neurologic adverse event had the most prominent proliferation of CD3⁺ T_{EM} cells (Figure 3B; OS ≥ 30 months without further treatment). Additionally, depletion kinetics of CD19⁺ B cells differed between patients with OS < 30 months and those with OS ≥ 30 months with further treatment (Figure 3C). Mean B-cell depletion was complete at day 8 and sustained throughout treatment cycles 1 and 2 in patients with OS ≥ 30 months. In contrast, in patients with OS < 30 months, mean B-cell depletion lasted 22 days and was not sustained through day 43 (the start of treatment cycle 2).

**Table 1. Clinical characteristics (long-term survivors)**

| | | | | Long-term Survivors (n = 10) |
|---|---|---|---|---|
| Age, years | | | | |
| | Median | | | 35 |
| | Range | | | (21-72) |
| Prior therapy/disease status, n (%) | | | | |
| | Prior allogeneic SCT | | | 4 (40) |
| | No prior allogeneic SCT | | | 6 (60) |
| | | Primary refractory | | 2 (20) |
| | | Salvage 1 after first complete remission | | 4 (40) |
| | | | ≤ 12 months after initial diagnosis | 2 (20) |
| | | | > 12 months after initial diagnosis | 2 (20) |
| | | ≥ 2^{nd} salvage | | 0(0) |
| Bone marrow blasts at screening, % | | | | |
| | Median | | | 56 |
| | Range | | | (8-97) |

| | | | | |
|---|---|---|---|---|
| **SCT, stem-cell transplantation. Long-term survivors were defined as patients with survival ≥ 30 months.** | | | | |

**Table 2. Responses and relapses (long-term survivors)**

| | | Long-term Survivors (n = 10) |
|---|---|---|
| Best overall response to blinatumomab, n (%) | | |
| | CR | 7 (70) |
| | CRh | 2 (20) |
| | Hypocellular bone marrow | 1 (10) |
| MRD response (< 10⁻⁴) to blinatumomab, n (%) | | 10 (100) |
| Allogeneic SCT administered after blinatumomab, n (%) | | |
| | Yes | 6 (60) |
| | No | 4 (40) |
| Relapse after blinatumomab, n (%) | | 3 (30) |
| | CD19-positive | 2 (20) |
| | CD19-negative | 1 (10) |
| | Retreatment with blinatumomab | 1 (10) |
| | Retreatment with chemotherapy | 2 (20) |

| | | |
|---|---|---|
| **CR, complete remission; CRh, CR with partial recovery of peripheral blood counts; SCT, stem cell transplantation;** **MRD, minimal residual disease. Long-term survivors were defined as patients with survival ≥ 30 months.** | | |

**Table 3. Neurologic events and cytokine release syndrome leading to treatment interruption**

| **Patient No.*** | **Blinatumomab Dose (µg/m²/day)** | **Cycle** | **Adverse Event, CTCAE grade** | **I Blinatumomab Dose at Restart of Treatment (µg/m²/day)** | **Prophylaxis** | **Response** | **Alive/In Remission^{†}** |
|---|---|---|---|---|---|---|---|
| 1 | 15 | 1 | Focal seizure grade 2 | 5 | clobazam | CRh | Yes/Relapse |
| | | | | | | RFS: 17.5 mo | |
| | | | | | | OS: 38.6 mo | |
| 4 | 30 | 2 | Encephalopathy grade 3 | 5 | No | CR | Yes/Yes |
| | | | | | | RFS: 34.1 mo | |
| | | | | | | OS: 36.9 mo | |
| 3 | 5 | 1 | CRS grade 4 | 5 | Prephase dexamethasone^{‡} | CRh | Yes/Yes |
| | | | | | | RFS: 22.4 mo | |
| | | | | | | OS: 30.0 mo | |

| | | | | | | | |
|---|---|---|---|---|---|---|---|
| **CR, complete remission; CRh, CR with partial recovery of peripheral blood counts; CRS, cytokine release syndrome; mo, months** **Long-term survivors were defined as patients with survival ≥ 30 months. "Patient No. refers to patients listed in Supplemental Table 1. ^{†}Alive at last follow-up/completed study in remission *Up to 24 mg dexamethasone per day for up to 5 days and/or 200 mg/m² cyclophosphamide per day for up to 4 days before blinatumomab infusion** | | | | | | | |

**Supplemental Table 1. Patient Characteristics of the 10 Long-term Survivors, Prephase Treatment and Response to Blinatumomab**

| | | | Patient Characteristics Before Blinatumomab Treatment | | | | | | Prephase Treatment | Response to Blinatumomab | | |
|---|---|---|---|---|---|---|---|---|---|---|---|---|
| Patient No. | Cohort* | Sex | Salvage or Primary Refractory | Time to Relapse, months | Prior SCT | Age, years | Blast Count, % | LDH, U/L | Dexamethasone/ Cyclophosphamide | CR/CRh | MRD Response | Relapse |
| 1 | 2b | Female | *NA* | 48.9 | Yes | 38 | 86 | 408 | Yes / Yes | Yes (CRh) | Yes | Yes (CD19+) |
| 2 | 3 | Female | Primary refractory | - | No | 29 | 9 | 217 | Yes / No | Yes | Yes | No |
| 3 | 3 | Male | Salvage 1 at relapse | 21.9 | No | 44 | 90 | 199 | Yes / No | Yes (CRh) | Yes | No |
| 4 | 2b | Female | Salvage 1 at relapse | 22.0 | No | 72 | 61 | 187 | No / No | Yes | Yes | No |
| 5 | 3 | Male | *NA* | 26.9 | Yes | 21 | 51 | 162 | Yes / Yes | Not | Yes | No |
| 6 | 3 | Male | Primary refractory | - | No | 57 | 19 | 161 | Yes / No | Yes | Yes | Yes (CD19+) |
| 7 | 3 | Male | *NA* | 23.9 | Yes | 21 | 8 | 299 | Yes / Yes | Yes | Yes | No |
| 8$ | 2a | Female | Salvage 1 at relapse | 6.4 | No | 66 | 8 | 246 | No / No | Yes (CRh) | Yes | Yes (CD19-) |
| 9 | 3 | Female | Salvage 1 at relapse | 8.5 | No | 32 | 78 | 772 | Yes / No | Yes | Yes | No |
| 10 | 3 | Male | *NA* | 29.0 | Yes | 29 | 97 | 135 | Yes / Yes | Yes | Yes | No |

| | | | | | | | | | | | | |
|---|---|---|---|---|---|---|---|---|---|---|---|---|
| CR, complete remission (≤ 5% blasts in the bone marrow, no evidence of circulating blasts or extra medullary disease, platelets > 100,000/µL, hemoglobin ≥ 11 g/dL, and absolute neutrophil count > 1,500/µL); CRh, CR with partial hematologic recovery of peripheral blood counts (≤ 5% blasts in the bone marrow, no evidence of circulating blasts or extra medullary disease, platelets > 50,000/µL, hemoglobin ≥ 7 g/dL, and absolute neutrophil count > 500/µL): SCT, stem cell transplantation; LDH, lactate dehydrogenase; MRD, minimal residual disease; NA, not applicable *Blinatumomab was administered by continuous infusion for 4 weeks with 2-week treatment-free intervals. in cohorts 2a and 3, patients received blinatumomab 5 µg/m²/day in the first week and then 15 µg/m²/day. in cohort 2b, patients received 5 µg/m²/day in the first week, 15 µg/m²/day in the second week, and then 30 µg/m²/day. ^{†}Hypocellular bone marrow ^{‡}Philadelphia chromosome-positive (protocol deviation) | | | | | | | | | | | | |

### DISCUSSION

This long-term follow-up analysis to the primary analysis of the first phase 2 study of blinatumomab in adult patients with r/r B-precursor ALL demonstrated a median OS of 13.0 months with 32.6 months of follow-up. Previous studies reported that chemotherapy results in median OS of 8.4 months or less in patients with r/r ALL. ¹⁻⁵ Recently published results for inotuzumab ozogamicin, an anti-CD22 monoclonal antibody drug conjugate, in r/r ALL reported a median OS of 7.3 months at a weekly dosing schedule.¹² One previous study reported that OS with salvage chemotherapy in relapsed ALL is shorter among patients who relapse after allogeneic SCT.⁵ In the present study, 15 of 36 patients (42%) had relapsed after allogeneic SCT before they received blinatumomab, but there was no difference in OS between patients with and without prior allogeneic SCT.

In this follow-up analysis, 10 of 36 patients (28%) were long-term survivors, defined as an OS of ≥ 30 months. All 10 long-term survivors had an MRD response, resulting in a long-term survival rate of 40% in patients with an MRD response. Although collection of larger data sets is warranted, the results indicate that achievement of an MRD response with blinatumomab treatment in r/r B-precursor ALL may translate into clinical benefit in terms of long-term survival.

Four long-term survivors did not receive allogeneic SCT as consolidation for blinatumomab. All four patients are still alive. One of the four patients had a CD19-negative relapse during the third cycle of blinatumomab, achieved another CR with chemotherapy, and received allogeneic SCT as consolidation for the chemotherapy. The other three patients received no other treatment after blinatumomab. All three patients experienced neurologic adverse events or CRS that resulted in interruption of blinatumomab treatment. Similar toxicities were reported in a phase 1 study of autologous T cells expressing the 19-28z chimeric antigen receptor (CAR) specific for the CD19 antigen,¹³ suggesting that toxicities against CD19-expressing target cells may be comparable and possibly independent of the mechanism of T-cell activation. The long-term outcomes of these three patients illustrate that long-term survival following blinatumomab treatment may be achieved with no other subsequent treatment even in relapsed ALL. Adverse events were managed by infusion interruption, and infusion restart was tolerated in all three patients. One patient without allogeneic SCT as consolidation for blinatumomab had a CD19-negative relapse during the third blinatumomab treatment cycle. This patient achieved another remission following chemotherapy. It is possible that the use of blinatumomab to prolong the interval between chemotherapy regimens enhanced sensitivity to the subsequent chemotherapy. Larger data sets are needed to confirm this observation. Patients with CD19-positive relapse after blinatumomab treatment may also be candidates for blinatumomab retreatment. One long-term survivor in this study who relapsed twice responded to retreatment with blinatumomab both times. Thus, blinatumomab treatment might be an alternative to chemotherapy for treatment of relapses after blinatumomab-induced CR followed by blinatumomab maintenance treatment. However, additional research is required to confirm the activity and tolerability of blinatumomab retreatment in this setting.

Long-term survivors had a significantly higher degree of T-cell and T_{EM}-cell expansion during treatment cycles 1 and 2. The data suggest that T-cell expansion might be a key factor for the antileukemia activity of blinatumomab in the setting of r/r ALL. The onset of antileukemic effects following blinatumomab infusion has been observed to occur early during treatment, in most cases by the end of the first cycle at the latest. For nonresponders, adding additional cycles of treatment or increasing the blinatumomab dose in the second cycle had little effect on improvement of antileukemic activity.⁹ In the current study, a dose increase to 30µg/m²/day on day 15 of the first cycle did not result in a higher CR rate (data not shown). Our study results suggest that T-cell expansion and kinetics of peripheral B-cell clearance might be important not only for remission but also for long-term OS. Overall survival of at least 30 months was associated with a greater T-cell expansion, compared with OS of less than 30 months. The greater T-cell expansion in the long-term survivors was associated with a complete clearance of B cells from peripheral blood within the first 7 days of treatment with blinatumomab. One might speculate that the degree of B-cell depletion within the first 7 days of treatment might serve as an early indicator for efficacy of blinatumomab, possibly permitting an early adjustment of the blinatumomab regimen via a dose increase.

In conclusion, in this long-term follow-up analysis of an exploratory, dose-escalation phase 2 study of blinatumomab in adult patients with r/r B-precursor ALL, an MRD response to blinatumomab was associated with significantly longer OS. Ten patients (28%) who received blinatumomab achieved an OS ≥ 30 months and were considered to be long-term survivors. All of the long-term survivors had an MRD response. Long-term survivors also had more rapid clearance of peripheral B cells and significantly greater T-cell expansion. The data suggest that long-term survival after blinatumomab treatment is associated with an MRD response and potentially also a high degree of T-cell expansion.

### REFERENCES

1. Tavernier E, Boiron JM, Huguet F, et al. Outcome of treatment after first relapse in adults with acute lymphoblastic leukemia initially treated by the LALA-94 trial. Leukemia. 2007;21(9):1907-1914.
2. Oriol A, Vives S, Hernández-Rivas JM, et al. Outcome after relapse of acute lymphoblastic leukemia in adult patients included in four consecutive risk-adapted trials by the PETHEMA Study Group. Haematologica. 2010;95(4):589-596.
3. Fielding AK, Richards SM, Chopra R, et al. Outcome of 609 adults after relapse of acute lymphoblastic leukemia (ALL); an MRC UKALL12/ECOG 2993 study. Blood. 2007; 109(3):944-950.
4. Kantarjian HM, Thomas D, Ravandi F, et al. Defining the course and prognosis of adults with acute lymphocytic leukemia in first salvage after induction failure or short first remission duration. Cancer. 2010;116(24):5568-5574.
5. Gökbuget N, Stanze D, Beck J, et al. Outcome of relapsed adult lymphoblastic leukemia depends on response to salvage chemotherapy, prognostic factors, and performance of stem cell transplantation. Blood. 2012;120(10):2032-2041.
6. Nagorsen D, Baeuerle PA. Immunomodulatory therapy of cancer with T cell-engaging BiTE antibody blinatumomab. Exp Cell Res. 2011;317(9): 1255-1260.
7. Topp MS, Gökbuget N, Zugmaier G, et al. Phase II trial of the anti-CD19 bispecific T cell-engager blinatumomab shows hematologic and molecular remissions in patients with relapsed or refractory B-precursor acute lymphoblastic leukemia. J Clin Oncol. 2014;32(36):4134-4140.
8. Topp MS, Gokbuget N, Stein AS, et al. Safety and activity of blinatumomab for adult patients with relapsed or refractory B-precursor acute lymphoblastic leukaemia: a multicentre, single-arm, phase 2 study. Lancet Oncol. 2015;16(1):57-66.
9. Topp MS, Kufer P, Gökbuget N, et al. Targeted therapy with the T-cell-engaging antibody blinatumomab of chemotherapy-refractory minimal residual disease in B-lineage acute lymphoblastic leukemia patients results in high response rate and prolonged leukemia-free survival. J Clin Oncol. 2011;29(18):2493-2498.
10. National Cancer Institute. NCI Common Terminology Criteria for Adverse Events (CTCAE) v4.0. Bethesda, MD; 2014.
11. Bargou R, Leo E, Zugmaier G, et al. Tumor regression in cancer patients by very low doses of a T cell-engaging antibody. Science. 2008;321(5891):974-977.
12. Kantarjian H, Thomas D, Jorgensen J, et al. Results of inotuzumab ozogamicin, a CD22 monoclonal antibody, in refractory and relapsed acute lymphocytic leukemia. Cancer. 2013;119(15):2728-2736.
13. Davila ML, Riviere I, Wang X, et al. Efficacy and toxicity management of 19-28z CAR T cell therapy in B cell acute lymphoblastic leukemia. Sci Transl Med. 2014;6(224):224ra225.
14. Brentjens RJ, Davila ML, Riviere I, et al. CD19-targeted T cells rapidly induce molecular remissions in adults with chemotherapy-refractory acute lymphoblastic leukemia. Sci Transl Med. 2013;5(177):177ra138.
15. Grupp SA, Kalos M, Barrett D, et al. Chimeric antigen receptor-modified T cells for acute lymphoid leukemia. N Engl J Med. 2013;368(16):1509-1518.
16. Kochenderfer JN, Dudley ME, Feldman SA, et al. B-cell depletion and remissions of malignancy along with cytokine-associated toxicity in a clinical trial of anti-CD19 chimeric-antigen-receptor-transduced T cells. Blood. 2012;119(12):2709-2720.

## Claims

1. A method of conserving or increasing the number of long term survivors in a group of patients suffering from ALL said method comprising administering a (therapeutically effective amount of) B cell depleting agent to said patients and/or adjusting the treatment regimen or dosage of a B cell depleting agent such that the number of B-cells in the blood of (preferably all of) the patients of said group remains or falls below one B cell/ml serum within a predefined period of time after the initial treatment with said B cell depleting agent.

2. A method of treating ALL in a patient or in a group of patients comprising administering a (therapeutically effective amount of) B cell depleting agent to said patients such that the number of B-cells in the blood of said patient or said group of patients remains or falls below one B cell/ml serum within a predefined period of time after the initial treatment with said B cell depleting agent.

3. A method of treating ALL in a patient or in a group of patients comprising (a) administering a B cell depleting agent to said patient or said group of patients, and (b) determining or monitoring whether the number of B-cells in the blood of said patient or in said group of patients remains or falls below one B cell/ml serum within a first predefined period of time after the initial treatment with said B cell depleting agent.

4. The method of claim 3 further comprising the step of (c) adjusting the B cell depleting agent such that the number of B-cells in the blood of said patient or said group of patients remains or falls below one B cell/ml serum within a predefined period of time after the initial treatment with said B cell depleting agent.

5. A B cell depleting agent for use in a method of treating ALL, wherein said B cell depleting agent is to be administered or adjusted such that the number of B-cells in the blood of the ALL patient remains or falls below one B cell/ml serum within a predefined period of time after the initial treatment with said B cell depleting agent.

6. A B cell depleting agent for use in a method of treating ALL, wherein said B cell depleting agent is to be administered or adjusted such that the number of B-cells in the blood of the ALL patient falls below one B cell/ml serum within a predefined period of time after the initial treatment with said B cell depleting agent.

7. A B cell depleting agent for use in a method of treating ALL in a group of ALL patients, said method comprising (a) administering a B cell depleting agent, and (b) determining or monitoring whether the number of B-cells in the blood of said patient remains or falls below one B cell/ml serum within a first predefined period of time after the initial treatment with said B cell depleting agent.

8. The B cell depleting agent of claim 7 further comprising the step of (c) adjusting the B cell depleting agent such that the number of B-cells in the blood of said ALL patient remains or falls below one B cell/ml serum within a predefined period of time after the initial treatment with said B cell depleting agent.

9. A B cell depleting agent for use in a method of conserving or increasing the number of long term survivors in a group of patients suffering from ALL, said method comprising administering a B cell depleting agent and/or adjusting the treatment regimen or dosage of a B cell depleting agent such that the number of B-cells in the blood of the patients of said group falls below one B cell/ml serum within a (first) predefined period of time after the initial treatment with said B cell depleting agent.

10. Use of the number of B cells in the blood of an ALL patient for adjusting the dosage or treatment regimen with a B cell depleting agent such that the number of B-cells in the blood of said patient remains or falls below one B cell/ml serum within a (first) predefined period of time after the initial treatment with said B cell depleting agent.

11. Use of a blood sample of an ALL patient for determining the number of B cells for adjusting the dosage or treatment regimen with a B cell depleting agent such that the number of B-cells in the blood of said patient remains or falls below one B cell/ml serum within a predefined period of time after the initial treatment with said B cell depleting agent.

12. A method of adjusting the dosage or treatment regimen with a B cell depleting agent in a patient suffering from ALL, said method comprising the step of observing the number of B-cells in the blood of said ALL patient within a predefined first period of time after the initial treatment with said B cell depleting agent and adjusting the dosage or treatment regimen such that the number of B-cells in the blood of said patient remains or falls below one B cell/ml serum within a predefined period of time after the initial treatment with said B cell depleting agent.

13. Any one of the preceding claims wherein said predetermined period of time is 15 days or less.

14. Any one of the preceding claims wherein said first period of time is between 2 and 14 days after the initial treatment with said B cell depleting agent.
